# EUROPEAN PATENT SPECIFICATION

(11) **EP 3 679 135 B1**
(45) Date of publication and mention of the grant of the patent: **19.04.2023**
(21) Application number: 18778782.5
(22) Date of filing: 07.09.2018
(51) Int. Cl.: C12N 15/10, C12Q 1/6806, C40B 70/00, C12Q 1/689

(54) **NORMALIZATION FOR SEQUENCING LIBRARIES**
NORMALISIERUNG ZUR SEQUENZIERUNG VON BIBLIOTHEKEN
NORMALISATION POUR BANQUES DE SÉQUENÇAGE

(30) Priority: 08.09.2017 US 201762555782 P; 06.11.2017 US 201762582162 P; 14.05.2018 US 201862671410 P
(43) Date of publication of application: 15.07.2020
(73) Proprietor: Psomagen, Inc., Rockville, MD 20850 (US)
(72) Inventor: APTE, Zachary, San Francisco California 94103 (US); RICHMAN, Jessica, San Francisco California 94103 (US); ALMONACID, Daniel, San Francisco California 94103 (US); MORALES, Eduardo, San Francisco California 94103 (US); ORTIZ, Rodrigo, San Francisco California 94103 (US); ORDENES, Nicolas, San Francisco California 94103 (US); JIMENEZ, Juan, San Francisco California 94103 (US); BRAVO, Cristian, San Francisco California 94103 (US); SCIOSCIA, Natalia, San Francisco California 94103 (US); OLIVARES, Eduardo, San Francisco California 94103 (US); LEON, Luis, San Francisco California 94103 (US); COVARRUBIAS, Paulo, San Francisco California 94103 (US); SEPULVEDA, Felipe, San Francisco California 94103 (US); MELIS, Felipe, San Francisco California 94103 (US)
(74) Representative: dompatent von Kreisler Selting Werner - Partnerschaft von Patent- und Rechtsanwälten mbB
(86) International application number: PCT/US2018/050092
(87) International publication number: WO 2019/051319

(56) References cited:
- WO-A2-2013/003489
- US-A1- 2016 032 363
- US-A1- 2017 073 730
- OLIVIA U MASON ET AL: "Metagenome, metatranscriptome and single-cell sequencing reveal microbial response to Deepwater Horizon oil spill", THE ISME JOURNAL: MULTIDISCIPLINARY JOURNAL OF MICROBIAL ECOLOGY, vol. 6, no. 9, 21 June 2012 (2012-06-21), pages 1715-1727, XP055519616, United Kingdom ISSN: 1751-7362, DOI: 10.1038/ismej.2012.59
- E. A. FRANZOSA ET AL: "Relating the metatranscriptome and metagenome of the human gut", PROCEEDINGS OF THE NATIONAL ACADEMY OF SCIENCES OF THE UNITED STATES OF AMERICA, vol. 111, no. 22, 19 May 2014 (2014-05-19) , pages E2329-E2338, XP055519622, ISSN: 0027-8424, DOI: 10.1073/pnas.1319284111
- YAN WEI LIM ET AL: "Metagenomics and metatranscriptomics: Windows on CF-associated viral and microbial communities", JOURNAL OF CYSTIC FIBROSIS, vol. 12, no. 2, 1 March 2013 (2013-03-01), pages 154-164, XP055519624, NL ISSN: 1569-1993, DOI: 10.1016/j.jcf.2012.07.009
- MEHTA BHAVIK ET AL: "Comparison between magnetic bead and qPCR library normalisation methods for forensic MPS genotyping", INTERNATIONAL JOURNAL OF LEGAL MEDICINE, SPRINGER VERLAG, DE, vol. 132, no. 1, 18 April 2017 (2017-04-18), pages 125-132, XP036392105, ISSN: 0937-9827, DOI: 10.1007/S00414-017-1591-9 [retrieved on 2017-04-18]

## Description

### TECHNICAL FIELD

The disclosure generally relates to genomics and molecular biology.

### BACKGROUND

Next Generation Sequencing (NGS) technologies (e.g., NGS platforms) can reduce the cost of DNA and/or other nucleic sequencing, improve the quality of information obtained, and/or improve the scalability of the sequencing processes. NGS technologies can facilitate sequencing of small to large numbers of DNA and/or other nucleic acid samples with high depth of analysis, which can allow detection and deciphering of precise target DNA sequences and/or other suitable sequences. Mixtures of different nucleic acids (e.g., different DNA nucleic acids; etc.) can be simultaneously analyzed, which can facilitate analysis of composition of complex mixtures (e.g., DNA and/or other nucleic acids extracted from a complex ecological community including microorganisms; etc.), and/or rare DNA sequence variants from a pool of conserved sequences (e.g., generation of rare mutations in a small number of cells of a large tissue, etc.). However, construction of sequencing libraries for NGS and/or other sequencing approaches can include library preparation processes (e.g., DNA manipulation, amplification, etc.) that can introduce a variety of biases (e.g., towards different targets such as DNA targets, towards ratios between targets, etc.). For example, overrepresented target molecules in a sequencing library can lead to a decrease in detection of other target molecules that are underrepresented. Additionally, the number of sequenced reads may not necessarily represent a direct proportion of the nucleic acid molecules (e.g., DNA Molecules) present in the library (e.g., sequenced in the same sequencing run) or in the original mix, which can present difficulties in generating absolute quantitative data (e.g., precise numbers or estimations of the composition of the original biological sample analyzed, etc.).

Further, NGS technologies and/or other suitable sequencing technologies can be used for amplicon-associated sequencing (e.g., analysis associated with a single or small number of gene regions, such as for identification of one or more microorganism taxa in a biological sample, etc.) or microbial community-associated sequencing (e.g., analysis associated with a microbial community and/or other suitable ecological communities of a biological sample, such as including a whole community of nucleic acids as opposed to analysis of a single gene amplicon, etc.). However, biases described herein can additionally or alternatively be associated with library preparation for such sequencing applications. US 2017/073730 discloses methods, compositions and kits for normalizing nucleic acid libraries.
WO 2013/003489 discloses methods for genome complexity reduction and polymorphism detection.
US 2016/032363 discloses methods for the diagnosis and treatment of inflammatory bowel diseases.
O. U. Mason et al., in The ISME Journal (2012) 6, 1715-1727 discloses metagenome, metatranscriptome and single-cell sequencing to reveal microbial response to Deepwater Horizon oil spill. E.A. Franzosa et al., in PNAS, vol. 111, no. 22 (2014), pages E2329-E2338 discloses relations between metatranscriptiome and metagenome of the human gut. Yan Wei Lim et al., Journal of Cystic Fibrosis, vol. 12, no. 2 (2013), 154-164 discloses metagenomics and metatranscriptomics in connection with CF-associated viral and microbial communities.

### BRIEF DESCRIPTION OF THE FIGURES

FIG. 1 includes a flowchart representation of variations of an embodiment of a method;
FIG. 2 includes a flowchart representation of variations of an embodiment of a method;
FIG. 3 includes a flowchart representation of variations of an embodiment of a method;
FIG. 4 includes a flowchart representation of variations of an embodiment of a method;
FIG. 5 includes a flowchart representation of variations of an embodiment of a method;
FIG. 6 includes a specific example of a graph representation associated with generating a normalized sequencing library;
FIG. 7 includes a specific example of a graph representation associated with generating a normalized sequencing library;
FIG. 8 includes a specific example of a graph representation associated with performing a magnetic-based normalization process;
FIG. 9 includes a specific example of a graph representation associated with performing a magnetic-based normalization process;
FIG. 10 includes a specific example of a visual representation associated with performing a magnetic-based normalization process;
FIG. 11 includes a specific example of a graph representation associated with performing a magnetic-based normalization process.
FIG. 12 includes a specific example of a graph representation associated with performing a magnetic-based normalization process.

### DESCRIPTION OF THE EMBODIMENTS

The following description of the embodiments is not intended to limit the embodiments, but rather to enable any person skilled in the art to make and use. The invention is defined by the claims.

### 1. Overview.

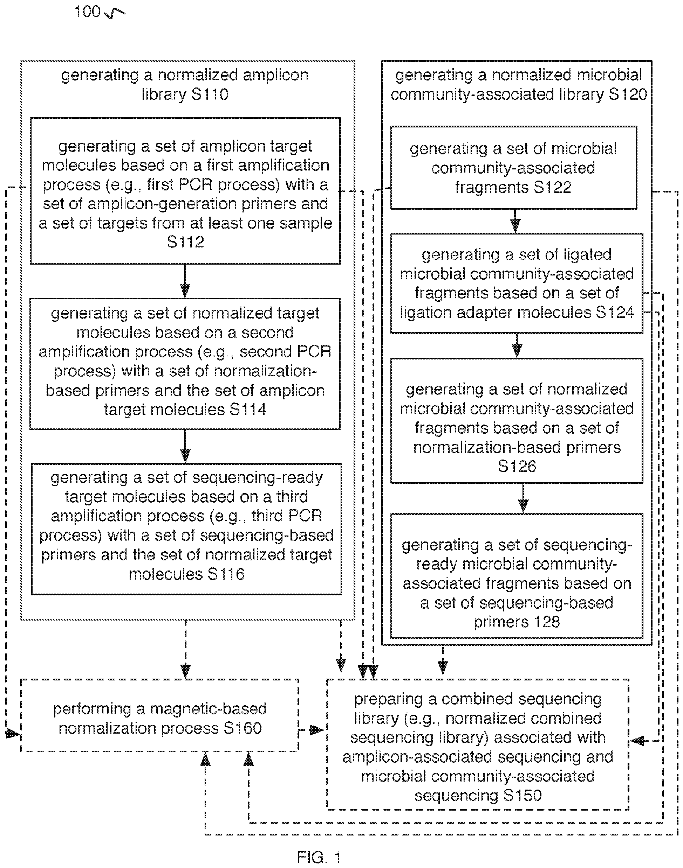

As shown in FIG. 1, embodiments of a method 100 (e.g., for preparation of a normalized library for sequencing, such as next-generation sequencing (NGS); preparation of a library associated with microorganisms, such as for sequencing microorganism nucleic acids; etc.) can include one or more of: generating one or more normalized amplicon libraries S110; and/or generating one or more normalized microbial community-associated libraries (e.g., normalized metagenome-associated libraries; normalized metatranscriptomic-associated libraries; etc.) S120.

In a specific example, the method 100 (e.g., for preparation of a normalized amplicon library for sequencing) can include one or more of: generating a set of amplicon target molecules based on a first amplification process with at least one sample and a set of amplicon-generation primers associated with a set of targets corresponding to target molecules from the at least one sample; generating a set of normalized target molecules based on a second amplification process with the set of amplicon target molecules and a set of normalization-based primers; and/or generating a set of sequencing-ready target molecules (e.g., normalized sequencing-ready target molecules suitable for NGS, etc.) based on a third amplification process with the set of normalized target molecules and a set of sequencing-based primers.

In a specific example, the method 100 (e.g., for preparation of a normalized microbial community-associated library for sequencing, etc.) can include one or more of: generating a set of microbial community-associated fragments from total nucleic acids of a sample, where the set of microbial community-associated fragments includes at least one of a set of metagenome-associated nucleic acid fragments and a set of metatranscriptomic-associated nucleic acid fragments; generating a set of ligated microbial community-associated fragments based on a ligation process with the set of microbial community-associated fragments and a set of ligation adapter molecules; generating a set of normalized microbial community-associated fragments based on a first amplification process with the set of ligated microbial community-associated fragments and a set of normalization-based primers; and/or generating a set of sequencing-ready microbial community-associated fragments (e.g., normalized sequencing-ready microbial community-associated fragments suitable for NGS, etc.) based on a second amplification process with the set of normalized microbial community-associated fragments and a set of sequencing-based primers, where the set of sequencing-ready microbial community-associated fragments includes at least one of a set of sequencing-ready metagenome-associated fragments and a set of sequencing-ready metatranscriptomic-associated fragments.

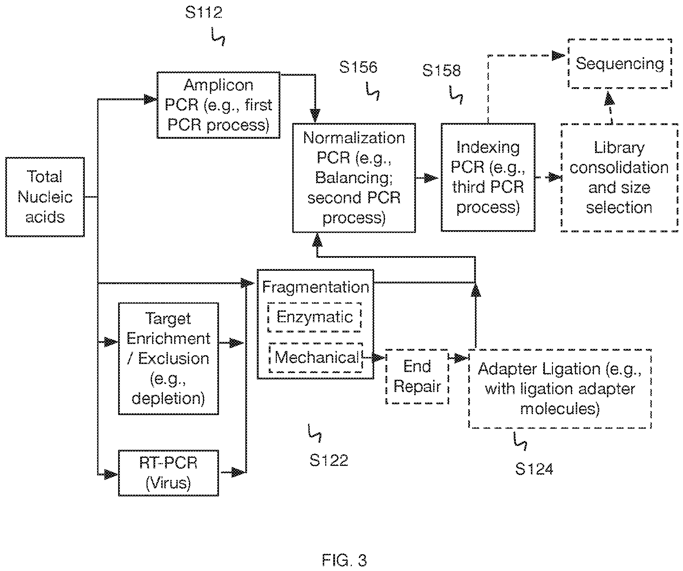

Additionally or alternatively, as shown in FIG. 1-3, embodiments of a method 100 can include generating a combined sequencing library (e.g., aggregate sequencing library), such as a combined sequencing library associated with (e.g., usable for; etc.) amplicon-associated sequencing and microbial community-associated sequencing (e.g., at least one of metagenome-associated sequencing and metatranscriptomic-associated sequencing; etc.) S150.

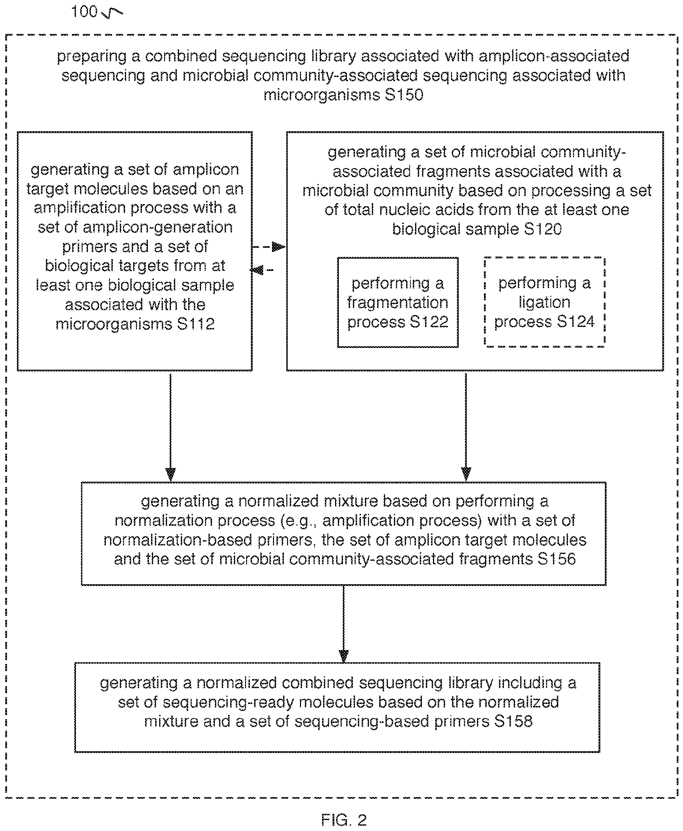

As shown in FIG. 2-3, embodiments of the method 100 (e.g., portions of embodiments of the method 100 including preparing a combined sequencing library S150, etc.) can additionally or alternatively include generating a normalized mixture based on performing a normalization process (e.g., a second amplification process, such as a second PCR process, such as where a first amplification process can be used for generating amplicon target molecules; etc.) with the set of amplicon target molecules and the set of microbial community-associated fragments S156; and/or generating a normalized combined library (e.g., including a set of sequencing-ready target molecules, etc.) based on the normalized mixture (e.g., based on a third amplification process with the normalized mixture; based on a third PCR process with the normalized mixture and the set of sequencing-based primers; etc.) S158.

In a specific example, the method 100 (e.g., for preparation of a normalized combined library for sequencing associated with a set of targets and a microbial community, etc.) can include one or more of: generating a set of amplicon target molecules based on a first amplification process with target molecules of at least one sample associated with microorganisms from the microbial community, where the target molecules correspond to the set of targets; generating a set of microbial community-associated fragments based on processing a set of total nucleic acids from at least one sample, where the set of microbial community-associated fragments includes at least one of a set of metagenome-associated nucleic acid fragments and a set of metatranscriptomic-associated nucleic acid fragments; generating a normalized mixture based on a second amplification process with the set of amplicon target molecules and the set of microbial community-associated fragments; and/or generating the normalized combined library including a set of sequencing-ready molecules based on a third amplification process with the normalized mixture, where the set of sequencing-ready molecules is associated with the set of targets and the microbial community.

Additionally or alternatively, embodiments of the method 100 can include performing an alternative or additional magnetic-based normalization process S160; processing (e.g., collecting; sample preparation for facilitating portions of embodiments of the method 100; performing portions of embodiments of the method 100 on; etc.) one or more biological samples from one or more users (e.g., subjects; humans; animals; patients; plants; etc.), such as biological samples collected from one or more body sites, which can include one or more of a gut site (e.g., as analyzed based on a stool sample, etc.), skin site, nose site, mouth site, genitals site, and/or other suitable physiological sites; adjusting concentrations of one or more subsets of primers (e.g., a subset of primers targeting a first target; etc.) relative other subsets of primers (e.g., a subset of primers targeting a second target; etc.) of a set of primers (e.g., for a particular amplification process described herein; etc.), such as for improving normalization results and/or other suitable purposes; determining microbiome characteristics (e.g., microorganism composition characteristics; microorganism function characteristics; characteristics associated with microorganism-related conditions, such as in relation to diagnosis and/or therapy; etc.) based on microorganism sequence datasets (e.g., microorganism sequence datasets generated based on sequencing with sequencing libraries generated from portions of embodiments of the method 100; microorganism sequence datasets generated from bioinformatic analysis associated with sequenced regions of sequencing libraries prepared as described herein; etc.). However, embodiments of the method 100 can additionally or alternatively include any suitable processes.

Embodiments of the method 100 and/or the system 200 can function to facilitate preparation of one or more normalized sequencing libraries (e.g., sequencing libraries with balanced output of targets from one or more samples, such as independent of initial input of targets, such as in relation to nucleic acid targets, etc.), such as including one or more of normalized amplicon libraries, normalized microbial community-associated libraries (e.g., normalized metagenome-associated libraries; normalized metatranscriptomic-associated libraries; etc.), normalized combined libraries (e.g., a normalized combined amplicon library and microbial community-associated library; a normalized aggregate sequencing library; etc.), and/or other suitable libraries; to facilitate absolute quantitation of molecules (e.g., target molecules, microbial community-associated fragments, etc.) for such libraries, such as through using unique molecular identifier (UMI)-based molecules (e.g., UMI-based primers; primers including UMI regions, such as amplicon-generation primers including UMI regions; ligation adapter molecules including UMI regions; etc.); and/or to facilitate other suitable library preparation-related and/or sequencing-related goals, such as in relation to microorganism-associated sequencing. Here we describe a simple, multiplexable and automatable protocol to obtain normalized amounts of varied DNA targets for their use in construction of balanced next generation sequencing libraries, both of the amplicon type and the metagenomic/metatranscriptomic type.

Additionally or alternatively, embodiments of the method 100 and/or system 200 can function to reduce biases associated with sequencing technologies (e.g., biases associated with conventional approaches of sequencing library preparation; biases affecting original ratios of individual molecules and/or target molecules from one or more original biological samples; biases associated with NGS technologies and/or other suitable sequencing technologies; etc.); improve quantitative analysis (e.g., analysis of absolute quantities; absolute quantitation of molecules, alleles, gene variants, and/or other components; etc.) of targets (e.g., nucleic acids; DNA molecules; nucleic acids in one or more original samples; etc.) associated with amplicon libraries and/or microbial community-associated libraries (e.g., metagenomic-associated libraries; metatranscriptomic-associated libraries; etc.), and/or other suitable components (e.g., through normalizing amounts of molecules based on specific multi-step amplification processes; etc.); improve processes associated with normalization of RNA transcripts (e.g., after RNA to DNA conversion; for metatranscriptomic-associated library preparation; etc.); improve automation (e.g., through automatable processes for facilitating normalization in relation to library preparation; etc.); improve normalization processes for different targets intra- and/or inter-sample, such as independent of initial template copy number; improve preparation of libraries including a plurality of targets in complex mixtures in parallel; and/or improve any suitable processes associated with library preparation and/or sequencing.

Additionally or alternatively, in variations, the method 100 and/or system 200 can function to improve automation, decrease cost, and facilitate normalization through improve application of magnetic particles and one or more magnetic field systems (e.g., configured to apply a magnetic field to magnetic particles bound to target molecules; etc.).

Additionally or alternatively, embodiments of the method 100 and/or system 200 can function to enable preparation of combined sequencing libraries, such as for facilitating performance of (e.g., combination of, etc.) amplicon-associated sequencing and microbial community-associated sequencing simultaneously (e.g., for sequencing with NGS technologies and/or other suitable sequencing technologies; etc.), such as to leverage the advantages (e.g., which can balance out disadvantages; which can facilitate new advantages of reducing analytical biases towards abundant microorganisms of a microbial community, of reducing requirements for degree of characterization of targets such as for primer design including conserved regions for the target and variable regions for differentiation from other taxa, such as in relation to taxonomic markers such as 16S rRNA, *rpoB,* and/or other markers; etc.) of both amplicon-associated sequencing and microbial community-associated sequencing (e.g., advantages of amplicon-associated sequencing, such as in enabling analysis of a large fraction of organisms in a microbial community that include a target gene and/or other target; advantages of microbial community-associated sequencing, such as in enabling unbiased analyses of microbial communities based on whole community DNA, such as in enabling characterization of a microbial community in relation to both microbiome composition, microbiome function, associated diversity and/or other suitable characteristics; etc.).

In a specific example, the method 100 can include generating combined amplicon (e.g., for taxonomic-related genes such as 16S, 18S, ITS, etc.) and microbial community-associated libraries (e.g., for enabling metagenomic detection of function-associated genes such as antibiotic genes, virulence genes, human genetic markers; for enabling detection of a plurality of RNA organisms such as viruses; for enabling detection of host and microbial transcribed genes, through mRNA, from a biological sample; metagenomic DNA library; etc.). In a specific example, the method 100 can include generating a broad target nucleic acid (e.g., DNA) library.

Additionally or alternatively, embodiments of the method 100 and/or system 200 can function to facilitate provision of data (e.g., microorganism sequence data, etc.) for directed taxonomic profiling (and/or other suitable composition-related analysis) of organisms in one or more biological samples, as well as to facilitate provision of data (e.g., microorganism sequence data, etc.) for genetic functional profiling (and/or other suitable function-related analysis) of the organisms (e.g., through microbial community-associated approaches such as metagenome-associated sequencing and/or metatranscriptomic-associated sequencing, etc.), such as in an additional or alternative manner to performing function-related analysis (e.g., determining microbiome functional features, etc.) based on a standard or known genome, such as for characterizing (e.g., diagnosing, analyzing, providing information regarding, etc.) and/or facilitating therapeutic intervention (e.g., providing therapies; providing therapy recommendations; etc.) for one or more microorganism-related conditions.

Additionally or alternatively, embodiments of the method 100 and/or system 200 can function to facilitate microorganism-related detection (e.g., taxonomic detection of organisms of a sample as well as the detection of genes present or expressed in the same sample; detection of organisms with conserved taxonomic genes in a directed fashion, and/or unbiasedly detecting other eukaryotes, prokaryotes, viral organisms, and/or other suitable microorganisms with characterized or non-previously characterized DNA in one or more biological samples; detection of new, unknown, and/or unidentified potential nucleic acid targets, such as by complementing enrichment based protocols such as amplification of specific targets or regions like 16S, 18S, ITS, or any other site-directed based technology, with normalization for facilitating reduced bias library preparation; detection, in an unbiased manner, of known or identified nucleic acid targets such as associated with antibiotic resistance, virulence factors molecular markers, and other suitable targets of interest, such as by complementing enrichment- or depletion-based protocols; etc.). However, embodiments of the method 100 and/or system 200 can include any suitable functionality.

Embodiments of the method 100 and/or system 200 preferably facilitates library preparation associated with NGS (e.g., NGS technologies). NGS can include any one or more of high-throughput sequencing (e.g., facilitated through high-throughput sequencing technologies; massively parallel signature sequencing, Polony sequencing, 454 pyrosequencing, Illumina sequencing, SOLiD sequencing, Ion Torrent semiconductor sequencing, DNA nanoball sequencing, Heliscope single molecule sequencing, Single molecule real time (SMRT) sequencing, Nanopore DNA sequencing, etc.), any generation number of sequencing technologies (e.g., second-generation sequencing technologies, third-generation sequencing technologies, fourth-generation sequencing technologies, etc.), amplicon-associated sequencing (e.g., targeted amplicon sequencing), microbial community-associated sequencing (e.g., metatranscriptomic sequencing, metagenomic sequencing, etc.), sequencing-by-synthesis, tunnelling currents sequencing, sequencing by hybridization, mass spectrometry sequencing, microscopy-based techniques, and/or any suitable NGS technologies.

Additionally or alternatively, embodiments of the method 100 and/or system 200 can facilitate library preparation and/or other suitable processes associated with any suitable sequencing (e.g., any suitable sequencing technologies, etc.), which can include any one or more of: capillary sequencing, Sanger sequencing (e.g., microfluidic Sanger sequencing, etc.), pyrosequencing, nanopore sequencing (Oxford nanopore sequencing, etc.), and/or any other suitable types of sequencing facilitated by any suitable sequencing technologies.

Embodiments of the method 100 and/or system 200 can improve sequencing library preparation for facilitating (e.g., based on microorganism sequence datasets derived from sequencing of the sequencing libraries; etc.) characterizations and/or therapies for one or more microorganism-related conditions, which can include one or more of: diseases, symptoms, causes (e.g., triggers, etc.), disorders, associated risk (e.g., propensity scores, etc.), associated severity, behaviors (e.g., caffeine consumption, habits, diets, etc.), and/or any other suitable aspects associated with microorganism-related conditions. Microorganism-related conditions can include one or more disease-related conditions, which can include any one or more of: gastrointestinal-related conditions (e.g., irritable bowel syndrome, inflammatory bowel disease, ulcerative colitis, celiac disease, Crohn's disease, bloating, hemorrhoidal disease, constipation, reflux, bloody stool, diarrhea, etc.); allergy-related conditions (e.g., allergies and/or intolerance associated with wheat, gluten, dairy, soy, peanut, shellfish, tree nut, egg, etc.); skin-related conditions (e.g., acne, dermatomyositis, eczema, rosacea, dry skin, psoriasis, dandruff, photosensitivity, etc.); locomotor-related conditions (e.g., gout, rheumatoid arthritis, osteoarthritis, reactive arthritis, multiple sclerosis, Parkinson's disease, etc.); cancer-related conditions (e.g., lymphoma; leukemia; blastoma; germ cell tumor; carcinoma; sarcoma; breast cancer; prostate cancer; basal cell cancer; skin cancer; colon cancer; lung cancer; cancer conditions associated with any suitable physiological region; etc.), cardiovascular-related conditions (e.g., coronary heart disease, inflammatory heart disease, valvular heart disease, obesity, stroke, etc.), anemia conditions (e.g., thalassemia; sickle cell; pernicious; fanconi; haemolyitic; aplastic; iron deficiency; etc.), neurological-related conditions (e.g., ADHD, ADD, anxiety, Asperger's syndrome, autism, chronic fatigue syndrome, depression, etc.), autoimmune-related conditions (e.g., Sprue, AIDS, Sjogren's, Lupus, etc.), endocrine-related conditions (e.g., obesity, Graves' disease, Hashimoto's thyroiditis, metabolic disease, Type I diabetes, Type II diabetes, etc.), Lyme disease conditions, communication-related conditions, sleep-related conditions, metabolic-related conditions, weight-related conditions, pain-related conditions, genetic-related conditions, chronic disease, and/or any other suitable type of disease-related conditions. Additionally or alternatively, microorganism-related conditions can include one or more human behavior conditions which can include any one or more of: caffeine consumption, alcohol consumption, other food item consumption, dietary supplement consumption, probiotic-related behaviors (e.g., consumption, avoidance, etc.), other dietary behaviors, habitué behaviors (e.g., smoking; exercise conditions such as low, moderate, and/or extreme exercise conditions; etc.), menopause, other biological processes, social behavior, other behaviors, and/or any other suitable human behavior conditions. Conditions can be associated with any suitable phenotypes (e.g., phenotypes measurable for a human, animal, plant, fungi body, etc.).

Embodiments of the method 100 and/or system 200 can be implemented for one or more biological samples from a single user, such as in relation to performing portions of embodiments of the method 100 for preparing a sequencing library from the one or more biological samples from the single user. Additionally or alternatively, embodiments can be implemented for biological samples from a set of users (e.g., population of subjects including the user, excluding the user, etc.), where the set of users can include subjects similar to and/or dissimilar to any other subjects for any suitable type of characteristics (e.g., in relation to microorganism-related conditions, demographic features behavior, microbiome composition and/or function, etc.); implemented for a subgroup of users (e.g., sharing characteristics, such as characteristics affecting portions of embodiments of the method 100; etc.); implemented for plants, animals, microorganisms (e.g., from environmental microbial communities; etc.), and/or any other suitable entities. Thus, information derived from a set of users (e.g., population of subjects, set of subjects, subgroup of users, etc.) can be used to provide additional insight for subsequent users (e.g., in relation to experimental parameters used in performing portions of embodiments of the method 100, etc.). In a variation, an aggregate set of biological samples can be associated with and processed for a wide variety of users, such as including users of one or more of: different demographics (e.g., genders, ages, marital statuses, ethnicities, nationalities, socioeconomic statuses, sexual orientations, etc.), different microorganism-related conditions (e.g., health and disease states; different genetic dispositions; etc.), different living situations (e.g., living alone, living with pets, living with a significant other, living with children, etc.), different dietary habits (e.g., omnivorous, vegetarian, vegan, sugar consumption, acid consumption, caffeine consumption, etc.), different behavioral tendencies (e.g., levels of physical activity, drug use, alcohol use, etc.), different levels of mobility (e.g., related to distance traveled within a given time period), and/or any other suitable characteristic (e.g., characteristics influencing, correlated with, and/or otherwise associated with microbiome composition and/or function, etc.), such as for comparing, for different types of users, amplicon-associated characteristics and microbial community-associated characteristics (e.g., where the amplicon-associated characteristics and microbial community-associated characteristics can be determined based on microorganism sequence datasets derived from combined sequencing libraries such as normalized combined sequencing libraries for simultaneous amplicon-associated sequencing and microbial community-associated sequencing, etc.). In examples, as the number of users increases, the predictive power of processes implemented in portions of embodiments of the method 100 can increase, such as in relation to characterizing a variety of users based upon their microbiomes (e.g., in relation to different collection sites for samples for the users, etc.). However, portions of embodiments of the method 100 and/or system 200 can be performed and/or configured in any suitable manner for any suitable entity or entities.

Microbial communities can include, relate to, and/or otherwise be associated with any suitable microorganisms of any suitable type (e.g., bacteria, archaea, fungi, protozoa, algae, viruses, etc.) and/or taxa, including at least one or more of:

Data described herein (e.g., data associated with normalization processes; data associated with amplification processes such as PCR processes; data associated with primers described herein; data associated with sequencing, such as sequencing reads, microorganism sequence datasets, and/or other suitable sequencing data; microbiome features; user data; supplementary data; data associated with microorganism-related conditions; etc.) can be associated with any suitable temporal indicators (e.g., seconds, minutes, hours, days, weeks, etc.) including one or more: temporal indicators indicating when the data was collected (e.g., temporal indicators indicating when a sample was collected; etc.), determined (e.g., temporal indicators indicating when sample processing operations were started, completed, etc.), transmitted, received, and/or otherwise processed; temporal indicators providing context to content described by the data; changes in temporal indicators (e.g., changes in outputs of sample processing operations over time, such as changes in products over cycles of PCR; etc.); and/or any other suitable indicators related to time. Molecules and/or any suitable biological components described herein can include any suitable size (e.g., sequence length, etc.).

Additionally or alternatively, parameters, metrics, inputs, outputs, and/or other suitable data can be associated with value types including any one or more of: scores, individual values, aggregate values, binary values, relative values, classifications, confidence levels, identifiers, values along a spectrum, and/or any other suitable types of values. Any suitable types of data, components (e.g., biological components), products (e.g., of sample processing operations, etc.), described herein can be used as inputs (e.g., for different sample processing operations such as different amplification processes; models; mixtures; sequencing technologies; etc.), generated as outputs (e.g., of different models; modules; products of sample processing operations; etc.), and/or manipulated in any suitable manner for any suitable components associated with embodiments of the method 100 and/or system 200.

One or more instances and/or portions of embodiments of the method 100 and/or processes described herein can be performed asynchronously (e.g., sequentially), concurrently (e.g., concurrent sequencing for a normalized combined sequencing library associated with amplicon-associated sequencing and microbial community-associated sequencing; multiplexing; processing a plurality of samples in portions of embodiments of the method 100; parallel data processing associated with sequencing analysis and/or portions of embodiments of the method 100; etc.), in temporal relation (e.g., substantially concurrently with, in response to, serially, prior to, subsequent to, etc.) to a trigger event (e.g., performance of a portion of an embodiment of the method 100), and/or in any other suitable order at any suitable time and frequency by and/or using one or more instances of the system 200, components, and/or entities described herein.

Primers described herein can be of any suitable size (e.g., and sequence length), can include any suitable primer regions described herein, and/or can be used with any suitable amplification processes described herein. Any suitable number and type of primers described herein include and/or otherwise be associated with any suitable number and type of primer regions described herein. Primer regions can include any suitable complementary versions of the primer regions (e.g., for facilitating annealing; amplification; etc.).

Additionally or alternatively, portions of embodiments of the method 100 and/or system 200 can facilitate (e.g., where outputs of portions of embodiments of the method 100 and/or system 200 can be subsequently used as inputs; etc.), improve, be used in conjunction with (e.g., serially, in parallel with, etc.), use (e.g., as inputs for portions of embodiments of the method 100 and/or system 200; etc.), have any suitable temporal relationship with, augment, modify, include, and/or can otherwise be associated with that described in US2017/0053061, US2017/0308669, US2019/0050534, US2018/0137240, US2018/0137239 and US2018/0362967.

However, the method 100 and/or system 200 can be configured in any suitable manner.

### 2.1 Generating a normalized amplicon library.

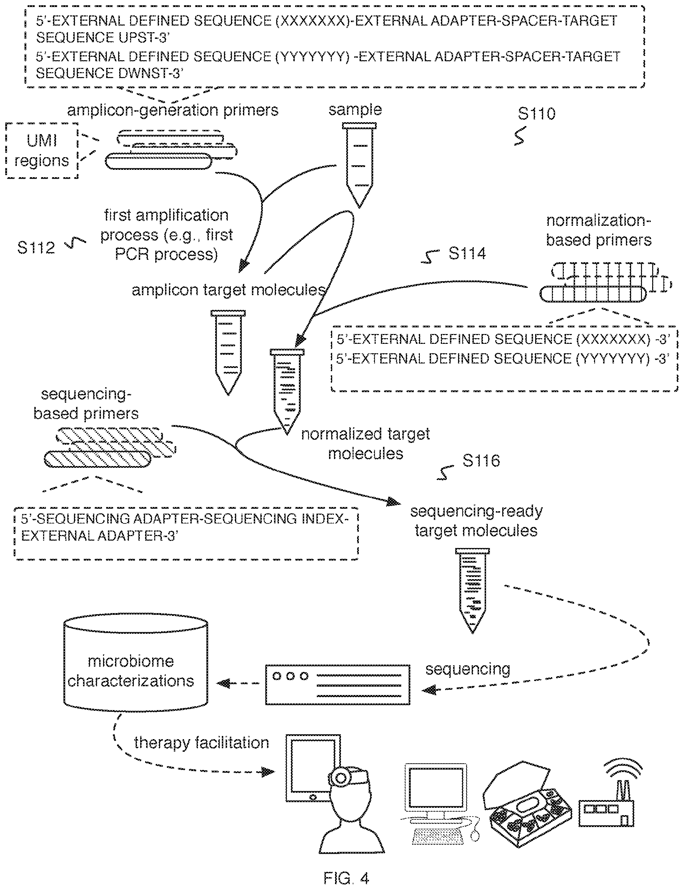

**As** shown in FIG. 1 and 4, embodiments of the method 100 can include generating one or more normalized amplicon libraries S110, which can function to prepare a normalized library for amplicon sequencing. Generating one or more normalized amplicon libraries S110 can additionally or alternatively include one or more of: generating a set of amplicon target molecules based on a first amplification process (a first PCR process; etc.) S112; generating a set of normalized target molecules based on a second amplification process a second PCR process; etc.) with the set of amplicon target molecules S114; generating a set of sequencing-ready target molecules (e.g., normalized sequencing-ready target molecules suitable for NGS, etc.) based on a third amplification process (a third PCR process; etc.) with the set of normalized target molecules S116 (e.g., using a set of sequencing-based primers including sequencing adapter regions for facilitating sequencing with a next-generation sequencing system; etc.); and/or other suitable processes for facilitating preparation of one or more normalized amplicon libraries.

Any suitable portions of generating a normalized amplicon library S110 can be performed in any suitable order and frequency relative each other. For example, generating amplicon target molecules S112 (and/or any suitable amplicons) can be performed at any suitable time and frequency (e.g., prior to generating normalized target molecules; during or after generating sequencing-ready target molecules, such as in an iterative product generation approach; before, concurrently with, and/or after processing microbial community-associated fragments for generation of a microbial community-associated library and/or combined library; and/or at any suitable time and frequency. However, any suitable portions of generating in any suitable order and/or frequency relative portions of embodiments of the method 100, and/or at any suitable time and frequency.

Generating a normalized amplicon library S110 can include any number of amplification processes (e.g., any number of PCR processes; multi-step PCR; etc.).

However, generating a normalized amplicon library S110 can be performed in any suitable manner.

### 2.1.A Generating a set of amplicon target molecules.

Embodiments of the method 100 can include generating a set of amplicon target molecules (e.g., from one or more biological samples associated with the one or more targets, such as biological samples including the one or more targets, etc.) S112, which can function to obtain amplicon target molecules for facilitating downstream normalization, other sample processing, and/or bioinformatics analyses.

Amplicon target molecules preferably include targets molecules (e.g., components including targets, such as total nucleic acids and/or nucleic acid fragments including target sequence regions, etc.) associated with (e.g., attached with; connected to; coupled with; etc.) one or more regions of amplicon-generation primers, but can additionally or alternatively include any suitable components associated with one or more targets associated with any suitable molecules. Generating the set of amplicon target molecules is preferably based on (e.g., using; process with; perform amplification processes with; etc.) a set of amplicon-generation primers and one or more biological samples (e.g., adding one or more regions of the amplicon-generation primers to one or more components, such as nucleic acids, of the one or more biological samples; etc.), but can additionally or alternatively be based on any suitable components.

Targets (e.g., targets of interest; known or identified targets; unknown or previously unidentified targets, etc.) can include any one or more of biomarkers; genes (e.g., gene expression markers, etc.); sequence regions (e.g., genetic sequences; sequences identifying a gene, chromosome, microorganism-related condition, conserved sequences, mutations, polymorphisms; amino acid sequences; nucleotide sequences; etc.); nucleic acids (e.g., genomic DNA, chromosomal DNA, extrachromosomal DNA, mitochondrial DNA, plastid DNA, plasmid DNA, cosmid DNA, phagemid DNA, synthetic DNA, cDNA obtained from RNA, single and double stranded DNA, etc.) cells; small molecules; proteins; peptides; targets associated with one or more microorganism-related conditions (e.g., targets informative of diagnosis, prognosis, prediction, and/or therapy associated with one or more microorganism-related conditions; etc.); targets associated with microorganism composition (e.g., targets indicative of taxonomic classification of microorganisms present in a sample; markers indicating presence, abundance, and/or absence of microorganisms of any suitable taxa; etc.) and/or microorganism function (e.g., targets indicative of functional features associated with microorganisms; etc.); lipids; total nucleic acids; whole microorganisms; metabolites; carbohydrates; and/or any suitable types of targets, such Target molecules can correspond to one or more targets, such as where target molecules can include, are of a type of, and/or are otherwise associated with one or more targets (e.g., where target nucleic acid molecules include the target sequence regions; etc.). Portions of embodiments of the method 100 can facilitate improved library preparation (e.g., normalized library preparation) to facilitate improved sequencing (e.g., NGS) and/or analysis of any suitable molecules.

Generating the set of amplicon target molecules is preferably based on (e.g., includes; uses outputs from; etc.) one or more amplification processes. Amplification processes (e.g., associated with generating the set of amplicon target molecules; associated with generating normalized molecules; associated with generating sequencing-ready molecules; associated with any suitable portions of embodiments of the method 100; etc.) preferably include one or more PCR processes (e.g., solid-phase PCR, RT-PCR, qPCR, multiplex PCR, touchdown PCR, nanoPCR, nested PCR, hot start PCR, etc.), but can additionally or alternatively include one or more of helicase-dependent amplification (HDA), loop mediated isothermal amplification (LAMP), self-sustained sequence replication (3SR), nucleic acid sequence based amplification (NASBA), strand displacement amplification (SDA), rolling circle amplification (RCA), ligase chain reaction (LCR), and/or any other suitable amplification processes.

Amplification processes associated with generating amplicon target molecules preferably use one or more amplicon-generation primers. For example, generating a set of amplicon target molecules can be based on an amplification process (e.g., a first amplification process of a set of amplification processes for facilitating preparation of normalized libraries) with at least one sample and a set of amplicon-generation primers associated with a set of targets corresponding to target molecules from the at least one sample. Amplicon-generation primers can include any one or more of: external defined sequence regions; adapter regions (e.g., including external adapter regions; etc.); spacer regions; target-associated regions; UMI regions; and/or any other suitable regions (e.g., sequence regions; etc.).

In a specific example, generating the set of amplicon target molecules can be based on an amplification process with a set of amplicon-generation primers including: first primers corresponding to a first primer type configuration including "5'-EXTERNAL DEFINED SEQUENCE (XXXXXXX)-EXTERNAL ADAPTER-SPACER-TARGET SEQUENCE UPST-3'", and second primers corresponding to a second primer type configuration including "5'-EXTERNAL DEFINED SEQUENCE (YYYYYYY) -EXTERNAL ADAPTER-SPACER-TARGET SEQUENCE DWNST-3'"; such as where "EXTERNAL DEFINED SEQUENCE" (and/or other external defined sequence regions of any suitable primers described herein, etc.) can correspond to a defined external sequence (e.g., represented by XXXXXXX in first primer type configuration, and by YYYYYYY in second primer type configuration, etc.) that is unique and different from that incorporated in primers targeting other nucleic acid sequences, where the EXTERNAL DEFINED SEQUENCE (and/or other external defined sequence regions) can be of any length and any defined sequence, such as long as it facilitates the subsequent amplification of the set of amplicon target molecules; such as where "EXTERNAL ADAPTER" (and/or other adapter regions of any suitable primers described herein, etc.) can correspond to any adapter sequence that is suitable for the construction and sequencing of NGS libraries and/or other suitable sequencing libraries, and can have varied lengths depending on the sequencing technology used; such as where "SPACER" (and/or any other suitable spacer regions of any suitable primer regions described herein, etc.) can correspond to any stretch of sequence region in between other regions, and which does not interact fully in complementary binding with target molecule sequence regions; such as where the "TARGET SEQUENCE" (and/or other suitable target-associated regions of any suitable primers described herein, etc.) can be the region that anneals flanking upstream (UPST) and downstream (DWNST) to the complementary target nucleic acid sequence, and that can allow polymerase enzyme to copy and amplify the target nucleic acid molecule (e.g., DNA, cDNA such as RNA copied to DNA), RNA, and any other suitable nucleic acid molecules; etc.), such as where N number of primers targeting N number of "TARGET SEQUENCE" regions with different "EXTERNAL DEFINED SEQUENCE" regions can be combined in a single PCR reaction.

In a specific example, target molecules can be amplified by PCR in a conventional thermal cycler using amplicon-generation primers, such as with DNA polymerase enzyme and between two to forty PCR cycles in a thermocycler machine or similar equipment, which can generate multiple copies of each target molecule with added adapter regions (e.g., external adapter sequences, etc.), external defined sequence regions, and/or other suitable regions of amplicon-generation primers.

Amplicon-generation primers (and/or other suitable molecules, such as primers and/or other molecules described herein) preferably include one or more external defined sequence regions. External defined sequence regions can include manually defined sequences, automatically (e.g., computationally) defined sequences, and/or any suitable sequences. External defined sequence regions can be of any suitable size. Same, similar (e.g., complementary to; annealable to; associated with; etc.), and/or different external defined sequence regions can be used within a set of primers (e.g., a set of amplicon-generation primers) for an amplification process, between sets of primers for different amplification processes (e.g., between a set of amplicon-generation primers and a set of normalization-based primers; etc.); and/or across any suitable primers. However, external defined sequence regions of amplicon-generation primers, of other suitable primers, and/or of any suitable molecules can be configured in any suitable manner.

Amplicon-generation primers (and/or other suitable molecules, such as primers and/or other molecules described herein) preferably include one or more target-associated regions. Target-associated regions preferably include sequence regions (e.g., genetic sequences, etc.) but can additionally or alternatively include any suitable types of components (e.g., any suitable components associated with targets, such as bindable to, coupleable to, connectable to, influencing, informing, modifying, and/or with any suitable relationship with targets; etc.). Target-associated regions are preferably associated with (e.g., with sequence complementarity to; targeting; amplifiable with; processable with; etc.) one or more targets (e.g., sequence regions of nucleic acid target molecules such as DNA, cDNA, RNA; other suitable components of nucleic acid targets; other suitable sequences; etc.). In an example, a target-associated region can include a DNA sequence annealable with a complementary target DNA sequence (e.g., of a nucleic acid target). Target-associated regions preferably enable polymerases (e.g., DNA polymerases) to copy and amplify nucleic acid targets and/or other suitable components, but target-associated regions can include any suitable functionality. Target sequence regions can include upstream and downstream versions for facilitating annealing to target nucleic acid molecules. Target-associated regions can include any suitable length (e.g., at least 15 bases in length; any suitable number of bases; etc.). Alternatively, amplicon-generation regions can exclude target-associated regions. However, target-associated regions of amplicon-generation primers, of other suitable primers, and/or of any suitable molecules can be configured in any suitable manner.

Amplicon-generation primers (and/or other suitable molecules, such as primers and/or other molecules described herein) can include one or more adapter regions. Adapter regions preferably include external adapter regions (e.g., where one adapter region can include one or more external adapter regions; etc.), which can include sequence regions (e.g., sequences, etc.) configured to facilitate sequencing library preparation (e.g., configured to facilitate construction and sequencing of NGS libraries; etc.), but external adapter regions can additionally or alternatively include any suitable components for facilitating sequencing. External adapter regions can include any suitable length (e.g., sequence length; any suitable number of bases; etc.) and/or any suitable sequence regions (e.g., any suitable combination of bases, etc.), which can be determined based on the type of sequencing (e.g., type of sequencing technology used; etc.). Alternatively, amplicon-generation primers (and/or other suitable molecules) can exclude adapter regions. However, adapter regions can be configured in any suitable manner.

In variations, amplicon-generation primers can include one or more UMI regions (e.g., UMI-based primers; where an amplicon-generation primer can include a single UMI region; where an amplicon-generation primer can include a plurality of UMI regions; etc.). In examples, a UMI region (e.g., of an amplicon-generation primer; of any suitable primer described herein; etc.) can include a set of random "N" bases (e.g., N deoxynucleotide bases), where each random "N" base is selected from any one of an "A" base, a "G" base, a "T" base, and a "C" base.

In a specific example, generating the set of amplicon target molecules can be based on an amplification process with a set of amplicon-generation primers including: first primers corresponding to a first primer type configuration including "5'-EXTERNAL DEFINED SEQUENCE (XXXXXXX)-EXTERNAL ADAPTER-UNIQUE MOLECULAR IDENTIFIER-TARGET SEQUENCE UPST-3", and second primers corresponding to a second primer type configuration including "5'-EXTERNAL DEFINED SEQUENCE (YYYYYYY) -EXTERNAL ADAPTER-UNIQUE MOLECULAR IDENTIFIER-TARGET SEQUENCE DWNST-3'"; such as where "UNIQUE MOLECULAR IDENTIFIER" (and/or other suitable UMI regions of any suitable primers described herein, etc.) can include a string of "N" deoxynucleotide bases (e.g., "N" being any random base between "A", "C", "T", and "G" nucleic bases, etc.) which can be continuous or separated by defined bases, and which can have a length in between 2 to 20 bases, depending on the amount of target molecules and/or variants that are desired to be quantified or differentiated, such as where longer UMI regions allow for a larger number of random base combinations and thus a larger set of unique identifiers. In a specific example, an amplification process can use DNA polymerase enzyme, amplicon-generation primers including UMI regions, and between two to three PCR cycles in a thermocycler machine or similar equipment, which can generate single copies of each target molecule with added UMI regions, adapter regions (e.g., external adapter sequences, etc.), external defined sequence regions, and/or other suitable regions of amplicon-generation primers.

"N" bases can be continuous (e.g., a string of "N" bases, etc.), separated (e.g., by defined bases; by any suitable sequence regions; etc.), and/or be located at any suitable sequence position of a primer and/or UMI-based molecule. UMI regions can include any suitable sequence length (e.g., at least 2 "N" bases; fewer than 21 "N" bases; any suitable number of "N" bases; etc.). UMI region sequence length can be determined based on an amount and/or type of targets to be processes (e.g., quantified, differentiated, etc.), such as where a longer UMI region can facilitate a larger number of random base combinations and a larger set of unique identifiers (e.g., to be used for analyzing a larger number of types of targets to be differentiated; to be used for analyzing samples including a large number of templates and/or gene variants; etc.). In an example, the UMI region can include a 4N UMI region (e.g., a UMI region including 4 "N" bases, etc.). In a specific example, the UMI region can include an 8N UMI region, such as for an amplification process of a 16S gene. Additionally or alternatively, UMI regions and/or UMI-based primers can be configured in any suitable manner described in and/or analogous to US2018/0362967. However, UMI regions and UMI-based primers can be configured in any suitable manner.

However, performing any suitable PCR processes and/or other amplification processes (e.g., in relation to generating the set of amplicon target molecules; in relation to any suitable portions of embodiments of the method 100; etc.) can be performed in any suitable manner.

In variations, generating a set of amplicon target molecules can include performing one or more fragmentation processes, ligation processes, and/or other suitable processes (e.g., in addition to or alternative to amplification-based processes, etc.) such as to add one or more regions of amplicon-generation primers to the one or more target molecules such as nucleic acid target molecules (and/or other suitable components of the one or more biological samples, etc.).

In variations, generating the set of amplicon target molecules (and/or suitable portions of embodiments of the method 100) can include performing one or more purification processes (e.g., to purify any suitable components; to remove any suitable components; etc.). In an example, generating the set of amplicon target molecules based on an amplification process (e.g., a PCR process) can include performing a purification process with products of the amplification process to remove the set of amplicon-generation primers (and/or to remove other suitable components, etc.) from the products of the amplification process. In examples, the method 100 can include performing a purification process for products obtained from any suitable amplification processes described herein. Purification processes can include any one or more of: silica-based DNA binding mini-columns, Solid Phase Reversible Immobilization (SPRI) magnetic beads (e.g., for upscaling and automation, etc.), precipitation of nucleic acids from the biological samples (e.g., using alcohol-based precipitation methods), liquid-liquid based purification techniques (e.g., phenolchloroform extraction), chromatography-based purification techniques (e.g., column adsorption), purification techniques involving use of binding moiety-bound particles (e.g., magnetic beads, buoyant beads, beads with size distributions, ultrasonically responsive beads, etc.) configured to bind nucleic acids and configured to release nucleic acids in the presence of an elution environment (e.g., having an elution solution, providing a pH shift, providing a temperature shift, etc.), and/or any suitable purification processes. In a specific example, magnetic beads can enable purification of small amounts of products of PCR processes, such as by electrostatic interaction of DNA with the carboxyl coated bead. In a specific example (e.g., as an alternative to, etc.), performing a purification process with magnetic beads can include using between 1:1 to 1:0.6 sample to bead volume ratio (e.g., where interaction of small DNA molecules with the beads is disfavored and unspecific products of sizes preferably 100 bp and below are eliminated, etc.), and/or any suitable ratios. In a specific example (e.g., as an alternative to, etc.), performing a purification process with magnetic beads can include using between 5 to 100 units of Exonuclease I, and/or any other single-strand DNA degrading enzyme, such as to be added to obtained products from any suitable PCR processes, such as to selectively degrade amplicon-generation primers and/or other suitable components (e.g., from the amplification process). In a specific example, performing the purification process with magnetic beads can include supplementing the process by adding 1 to 100 units of DpnI restriction enzyme and/or other suitable enzyme, such as to degrade PCR template DNA and/or other suitable components. In a specific example, the combination of both enzymatic treatments and/or other suitable processes can be used in addition to or as an alternative to PCR product cleanup approaches. Additionally or alternatively, purification processes can be performed in any suitable manner (e.g., in relation to any suitable portions of embodiments of the method 100, etc.).

However, generating amplicon target molecules can be performed in any suitable manner.

### 2.1.B Generating normalized target molecules.

Embodiments of the method 100 can include generating a set of normalized target molecules (e.g., from an amplification process with amplicon target molecules; etc.) S114, which can function to obtain normalized target molecules for facilitating preparation of a normalized sequencing library, such as for normalizing representation of different targets in the sequencing library. In specific examples, generating normalized target molecules can function to improve equalization of amounts of different types of target molecules initially represented in the sample (e.g., improve representation of underrepresented targets; etc.).

Normalized target molecules preferably include amplicon target molecules (e.g., generated based on a first amplification process, etc.) associated with (e.g., attached with; connected to; coupled with; etc.) one or more regions of normalization-based primers, but can additionally or alternatively include any suitable components. Generating the set of normalized target molecules is preferably based on (e.g., using; process with; perform amplification processes with; etc.) a set of normalization-based primers and a set of amplicon target molecules, but can additionally or alternatively be based on any suitable components.

Generating the set of normalized target molecules is preferably based on (e.g., includes; uses outputs from; etc.) one or more amplification processes (e.g., PCR processes; other suitable types of amplification processes described herein; etc.), but can additionally or alternatively be based on any suitable type of sample processing operations (e.g., ligation operations; purification operations; etc.)

Amplification processes associated with generating normalized target molecules preferably use one or more normalization-based primers. For example, generating a set of normalized target molecules can be based on an amplification process (e.g., a second amplification process of a set of amplification processes for facilitating preparation of normalized libraries) with a set of amplicon target molecules and a set of normalization-based primers. Normalization-based primers preferably include external defined sequence regions (e.g., including any suitable characteristics described herein in relation to external defined sequence regions), but can additionally or alternatively include any other suitable primer regions.

In a specific example, generating the set of normalized target molecules can be based on an amplification process with a set of normalization-based primers (e.g., and a set of amplicon target molecules, such as to be used with the amplification process and/or other suitable amplification processes described herein; etc.) including: first primers corresponding to a first primer type configuration including "5'-EXTERNAL DEFINED SEQUENCE (XXXXXXX) -3'", and second primers corresponding to a second primer type configuration including "5'-EXTERNAL DEFINED SEQUENCE (YYYYYYY) -3'"; such as where "EXTERNAL DEFINED SEQUENCE" (and/or other external defined sequence regions of any suitable primers described herein, etc.) can correspond to a defined external sequence (e.g., represented by XXXXXXX in first primer type configuration, and by YYYYYYY in second primer type configuration, etc.), such as sequences of defined external sequence regions of amplicon-generation primers, such as where the "EXTERNAL DEFINED SEQUENCE" (and/or other external defined sequence regions) can be of any length and any defined sequence. A defined number of molecules of normalization-based primers can be added to the one or more amplification processes for generating normalized target molecules; and/or N number of cycles of PCR (and/or any suitable extent of amplification processes; etc.) can be performed to ensure that all added normalization-based primers are completely used (e.g., with DNA polymerase enzyme, etc.); such as where the number of amplicons, specific to each target nucleic acid sequence (e.g., of one or more target molecules; etc.), that can be generated from the one or more amplification processes can be limited to the number of normalization-based primer molecules added (e.g., independent of the number of amplicon target molecules used as a template for the one or more amplification processes. In examples, external defined sequence regions of normalization-based primers can anneal to external defined sequence regions of amplicon target molecules (e.g., external defined sequence regions added to target molecules, such as based on a first amplification process; etc.), such as where the external defined sequence regions of the normalization-based primers can be associated with (e.g., sharing sequences with; complementary to; annealable to; etc.) external defined sequence regions of amplicon target molecules; etc.). In a specific example, the set of amplicon-generation primers can include first external defined sequence regions, where generating the set of amplicon target molecules can include adding the first external defined sequence regions to the target molecules based on the first amplification process; and/or where the set of normalization-based primers can include second external defined sequence regions associated with (e.g., sharing sequences with; complementary to; annealable to; etc.) the first external defined sequence regions. In a specific example, the set of amplicon-generation primers can include target-associated regions for annealing to target sequence regions of the target molecules; and/or where generating the set of normalized target molecules can include, for the second amplification process, adding a defined amount of the set of normalization-based primers for annealing between the first and the second external defined sequence regions.

In specific examples, with normalization-based primers, even if a given amplicon target molecule (and/or targets of the sample) are over or underrepresented, such as prior to generation of the normalization target molecules; the generated normalized target molecules can reach desired amounts (e.g., upon completion of an associated PCR process; etc.), such as based on the amount of each specific primer type (e.g., corresponding to external defined sequence regions; etc.) used.

In variations, generating a set of normalized target molecules can include performing one or more fragmentation processes, ligation processes, and/or other suitable processes (e.g., in addition to or alternative to amplification-based processes, etc.) such as to add one or more regions of normalization-based primers to the one or more target molecules such as nucleic acid target molecules (and/or other suitable components of the one or more biological samples, etc.).

**In** variations, generating the set of normalized target molecules (and/or suitable portions of embodiments of the method 100) can include performing one or more purification processes (e.g., to purify any suitable components; to remove any suitable components; etc.). In an example, generating the set of normalized target molecules based on an amplification process (e.g., a PCR process) can include performing a purification process with products of the amplification process to remove the set of normalization-based primers (and/or to remove other suitable components, etc.) from the products of the amplification process. Any suitable purification processes described herein can be used, and/or can be performed in any suitable manner.

However, generating normalized target molecules can be performed in any suitable manner.

### 2.1.C Generating sequencing-ready target molecules.

Embodiments of the method 100 can include generating a set of sequencing-ready target molecules (e.g., NGS-ready normalization target molecules; based on the set of normalization target molecules and the set of sequencing-based primers; etc.) S116, which can function to process target molecules (e.g., normalization target molecules) for preparation for sequencing (e.g., NGS, etc.).

Preparing molecules for sequencing preferably includes preparing normalization target molecules for sequencing (e.g., by adding one or more adapter regions and/or one or more index regions, etc.), but can additionally or alternatively include preparing any suitable molecules for sequencing.

Generating the set of sequencing-ready target molecules is preferably based on (e.g., use; process with; perform amplification processes with; etc.) a set of normalization target molecules and a set of sequencing-based primers (e.g., for incorporation of the sequencing-based primers and/or any suitable regions of sequencing-based primers with the set of normalization target molecules; for adding regions of the sequencing-based primers to the set of normalization target molecules; etc.), but can additionally or alternatively be based on any suitable components. In an example, amplicon-generation primers can include first external adapter regions (e.g., associated with the NGS; etc.), where the set of normalization target molecules (e.g., derived from amplicon target molecules generated based on the amplicon-generation primers; etc.) includes the first external adapter regions; and where generating a set of sequencing-ready target molecules (e.g., NGS-ready normalization target molecules; etc.) includes annealing a set of sequencing-based primers (e.g., including an adapter region including external adapter regions, such as complementary external adapter regions to the first external adapter regions, etc.) with the normalization target molecules at the external adapter regions of the normalization target molecules (e.g., which can be common to all normalization target molecules, independent of the initial nucleic acid sequence of the target molecules; etc.). In an example, sequencing-based primers can include one or more adapter regions (e.g., sequencing adapter regions associated with the sequencing, such as NGS; external adapter regions annealable to normalization target molecules; etc.) and one or more index regions configured to facilitate multiplexing associated with the NGS; and where generating the set sequencing-ready target molecules includes adding the one or more index regions and the one or more adapter regions to the set of normalization target molecules, based on the amplification process (e.g., a third PCR process; etc.) with the normalization target molecules and the set of sequencing-based primers. Generating sequencing-ready target molecules preferably increases the concentration of normalized target molecules to an amount suitable for NGS and/or other suitable sequencing (e.g., to an amount at or above 2 pM and/or any suitable desired amount; etc.), such as where the sequencing-ready target molecules are at desired amounts (e.g., normalized amounts and suitable amounts for NGS, etc.).

In a specific example, performing a PCR process (e.g., a third PCR process for generating the set of sequencing-ready target molecules) can include using between and/or including 0.02-0.08 units/uL (and/or other suitable concentration) of polymerase (e.g., DNA polymerase), for between and/or including 15-45 cycles of PCR (and/or any suitable number of cycles of PCR). In a specific example, performing the PCR process (e.g., a third PCR process, etc.) can enable the amplification of clean, normalized nucleic acid products from the set of normalization target molecules (e.g., products from performing a second PCR process, etc.), which can increase the concentration of nucleic acid targets (e.g., target molecules) to levels suitable for sequencing (e.g., NGS; such as at least 2 pM and/or any suitable threshold amount).

In a specific example, generating the set of sequencing-ready target molecules can be based on an amplification process with a set of sequencing-based primers (e.g., and a set of normalized target molecules, such as to be used with the amplification process and/or other suitable amplification processes described herein; etc.) corresponding to a primer type configuration including "5'-SEQUENCING ADAPTER-SEQUENCING INDEX-EXTERNAL ADAPTER-3‴; such as where "SEQUENCING INDEX" (and/or other index regions of any suitable primers described herein, etc.) can correspond to a defined barcode sequence in between 2 to 10 bases long (and/or other suitable length) that can allow for combinatorial tagging of different samples that are going to be sequenced; such as where "SEQUENCING ADAPTER" (and/or other adapter regions of any suitable primers described herein, etc.) can correspond to any specific sequence that is required by and/or otherwise associated with one or more NGS technologies and/or other suitable sequencing technologies, such as for enabling sequencing to occur.

In variations, generating the set of sequencing-ready target molecules (and/or suitable portions of embodiments the method 100) can additionally or alternatively include performing one or more supplementary amplification processes (e.g., a fourth amplification process; a fourth PCR process; which can function to increase concentrations of sequencing-ready target molecules, of products of a third amplification process, and/or any other suitable components, etc.). In an example, the method 100 can including performing a supplementary PCR process (e.g., a fourth PCR process, where generating the amplicon target molecules includes performing a first PCR process, generating the normalization target molecules includes performing a second PCR process, and where generating the set of sequencing-ready target molecules includes performing a third PCR process; etc.), such as based on (e.g., using, with, etc.) primers annealing at sequencing adapter regions added by a PCR process (e.g., a second PCR process, etc.) and/or other suitable process used in generating the set of sequencing-ready target molecules. In a specific example, the set of amplicon-generation primers can include first adapter regions associated with the sequencing; where generating the set of amplicon target molecules can include adding the first adapter regions to the target molecules based on a first amplification process (e.g., a first PCR process; etc.); where the set of normalized target molecules can include the added first adapter regions; and/or where the set of sequencing-based primers can include second adapter regions for annealing to the added first adapter regions of the set of normalized target molecules.

Additionally or alternatively, annealing can occur at any suitable primer regions (e.g., at sequencing adapter regions added at any suitable amplification processes; etc.). In a specific example, performing a supplementary PCR process can be based on (e.g., in response to; triggered by; conditioned upon; etc.) concentrations (e.g., product concentrations; concentration of products from generating the set of sequencing-ready target molecules; products from a third PCR process; etc.) satisfying a threshold condition (e.g., concentration below 1 pM, etc.). In variations, generating the set of sequencing-ready target molecules (and/or suitable portions of embodiments the method 100) can additionally or alternatively include increasing concentrations of sequencing-ready target molecules and/or other suitable molecules based on sample binding to in silica columns, and/or elution in smaller volumes. In a specific example generating the set of sequencing-ready target molecules includes increasing the concentration of products of the third amplification process (e.g., for generating sequencing-ready target molecules; etc.), where increasing the concentration of the products of the third amplification process includes at least one of: performing a fourth amplification process based on products of the third amplification process and additional primers for annealing to adapter regions of the sequencing-based primer; and performing sample binding to silica columns with elution in smaller volumes.

Sequencing-based primers (and/or other suitable molecules described herein) preferably include one or more adapter regions. Adapter regions of sequencing-based primers preferably include one or more sequencing adapter regions, which preferably include sequence regions facilitative of NGS (e.g., sequence regions required by one or more NGS technologies for performing sequencing; sequence regions determined based on a type of NGS technology used; facilitate of NGS technologies; etc.) and/or other suitable sequencing technologies, but sequencing adapter regions can be configured in any suitable manner. Additionally or alternatively, any suitable adapter regions can include sequencing adapter regions. Adapter regions (e.g., of sequencing-based primers, etc.) preferably include one or more external adapter regions (e.g., same, similar to, different, complementary to external adapter regions of other adapter regions, such as adapter regions of amplicon-generation primers, etc.), but any suitable adapter regions can include the one or more external adapter regions. Adapter regions of sequencing-based primers preferably include one or more index regions (e.g., sequencing index region; etc.), which are preferably configured to facilitate multiplexing, combinatorial tagging of different samples (and/or components of samples, components to be sequences), and/or other suitable functionality associated with NGS and/or other sequencing. An index region preferably includes a defined barcode sequences (e.g., including a length of at least 2 bases and fewer than 11 bases; including a length of any suitable number of bases; etc.), but can additionally or alternatively include any suitable components including any suitable length. In a specific example, sequencing-based primers can include a configuration including "s'-SEQUENCING ADAPTER-SEQUENCING INDEX-EXTERNAL ADAPTER-3'". Adapter regions can include sequencing adapter regions separated from, contiguous with, and/or otherwise positioned relative external adapter regions, but any suitable regions can include any suitable positions relative other regions, and/or any suitable positions. Additionally or alternatively, sequencing-based primers can include any suitable regions (e.g., described herein in relation to primers, etc.) and/or other suitable components. However, sequencing-based primers can be configured in any suitable manner.

In a specific example, sequencing-ready target molecules (and/or suitable products of a normalized amplicon library) can include a configuration including: "5'-SEQUENCING ADAPTER- SEQUENCING INDEX- EXTERNAL ADAPTER-SPACER-TARGET SEQUENCE-SPACER-EXTERNAL ADAPTER-SEQUENCING INDEX-SEQUENCING ADAPTER-3'". In a specific example, such as where amplicon-generation primers including UMI regions are used (e.g., in generating amplicon target molecules, etc.), sequencing-ready target molecules (and/or suitable products of a normalized amplicon library) can include a configuration including "5'-SEQUENCING ADAPTER- SEQUENCING INDEX- EXTERNAL ADAPTER-UNIQUE MOLECULAR IDENTIFIER-TARGET-SEQUENCE-UNIQUE_MOLECULAR_IDENTIFIER-EXTERNAL ADAPTER-SEQUENCING INDEX-SEQUENCING ADAPTER-3'".

However, preparing a set of sequencing-based primers can be performed in any suitable manner.

However, generating the set of sequencing-ready target molecules can be performed in any suitable manner.

### 2.2 Generating a Normalized Microbial Community-Associated Library.

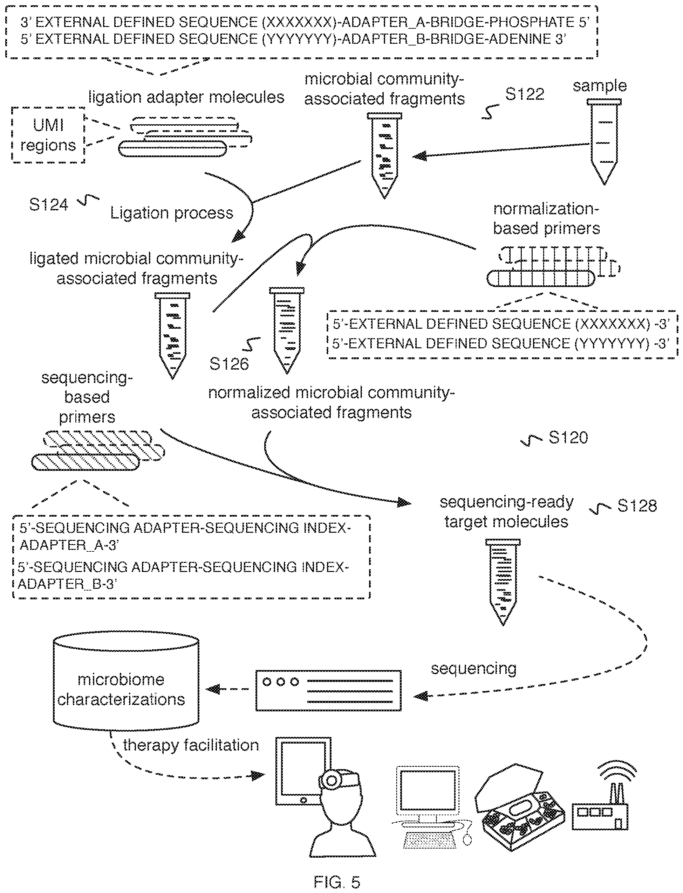

As shown in FIG. 1 and 5, embodiments of the method 100 can include generating one or more normalized microbial community-associated libraries (e.g., normalized metagenome-associated libraries; normalized metatranscriptomic-associated libraries; etc.) S120, which can function to prepare one or more normalized libraries for metagenome-associated sequencing and/or metatranscriptomic-associated sequencing.

Generating one or more normalized microbial community-associated libraries S120 can additionally or alternatively include one or more of: generating a set of microbial community-associated fragments from total nucleic acids of a sample S122; generating a set of ligated microbial community-associated fragments based on a ligation process with the set of microbial community-associated fragments S124; generating a set of normalized microbial community-associated fragments based on a first amplification process with the set of ligated microbial community-associated fragments S126; generating a set of sequencing-ready microbial community-associated fragments (e.g., normalized sequencing-ready microbial community-associated fragments suitable for NGS, etc.) based on a second amplification process with the set of normalized microbial community-associated fragments S128; and/or other suitable processes for facilitating preparation of one or more normalized microbial community-associated libraries. However, generating normalized microbial community-associated libraries can be performed in any suitable manner.

### 2.2.A Generating a set of microbial community-associated fragments.

Embodiments of the method 100 can include generating a set of microbial community-associated fragments (e.g., including at least one of a set of metagenome-associated fragments and/or a set of metatranscriptomic-associated fragments, etc.) S122, which can function to generate fragments facilitative of microbial community-associated sequencing (e.g., metagenome-associated sequencing and/or metatranscriptomic-associated sequencing; etc.). For example, generating a set of microbial community-associated fragments can be based on processing a set of total nucleic acids (and/or other suitable components, etc.) from one or more samples (e.g., biological samples, such as where any suitable biological samples can be collected at one or more body sites including any one or more of skin sites, genital sites, nose sites, gut sites, genital sites, etc.).

Microbial community-associated fragments can include raw fragments of total nucleic acids (e.g., products of performing fragmentation processes on total nucleic acids of one or more biological samples, etc.), processed fragments of total nucleic acids (e.g., processed with any suitable sample processing operations; fragments of pre-processed total nucleic acids and/or other suitable components; purified fragments; etc.) and/or any suitable fragments of total nucleic acids and/or other suitable components of one or more samples.

Microbial community-associated fragments are preferably associated with one or more microbial communities. A microbial community preferably includes microorganisms (e.g., sharing a common living space, such as a physiological region of a user, such as a sample collection site of a user; etc.) from a plurality of taxa (e.g., taxa including kingdoms, phyla, classes, orders, families, genera, species, subspecies, strains, and/or any other suitable groups of microorganisms; etc.), but can alternatively include only microorganisms from a single taxa. Additionally or alternatively, microbial communities can include interactions between microorganisms, products of interactions between microorganisms, relationships between microorganisms, functional features (e.g., functional profiles, etc.) associated with the microorganisms and/or microbial community, composition features (e.g., taxonomic profiles, etc.) associated with the microorganisms and/or microbial community, and/or any other suitable components and/or features associated with microorganisms and/or microbial communities.

Generating the set of microbial community-associated fragments is preferably based on processing a set of total nucleic acids, but can additionally or alternatively be based on processing any suitable components (e.g., nucleic acid fragments, targets such as nucleic acid targets, other suitable components, etc.).

Generating the set of microbial community-associated fragments (e.g., processing the set of total nucleic acids) preferably includes performing one or more fragmentation processes (e.g., fragmenting; generating fragments of; etc.) with total nucleic acids from the set of total nucleic acids (e.g., all or a subset of the set of total nucleic acids from one or more samples, etc.), but can additionally or alternatively include any suitable processes for facilitating microbial community-associated fragment generation. Fragmentation processes can include random fragmentation (e.g., to generate a randomized set of microbial community-associated fragments), semi-random fragmentation, and/or directed fragmentation (e.g., for generating a desired set of fragments; etc.). Performing one or more fragmentation processes (e.g., in relation to generating a set of microbial community-associated fragments; in relation to any suitable portions of embodiments of the method 100; etc.) can include any one or more of enzymatic processes (e.g., using non-sequence-specific DNA endonucleases such as DNAse I, using DNA nickases, a mix of restriction endonucleases, transposase-type enzymes, etc.), mechanical processes (e.g., sonication; nebulization; shearing such as hydrodynamic shearing; etc.), chemical processes (e.g., using a mix of hydroxyl-radical forming reagents such as iron-EDTA and/or other reagents; etc.), and/or any suitable types of fragmentation processes. However, performing one or more fragmentation processes (e.g., one or more enzymatic processes; one or more mechanical processes; etc.) can be performed in any suitable manner.

Generating the set of microbial community-associated fragments can additionally or alternatively include processing the set of total nucleic acids and/or the set of microbial community-associated fragments (e.g., prior to, during, and/or after performing one or more fragmentation processes; iteratively with performing fragmentation processes; etc.). Processing (e.g., the set of total nucleic acids; fragments; any suitable components), can include any one or more of transforming nucleic acids (e.g., transforming mRNA into cDNA), performing target-capture processes (e.g., enrichment processes, exclusion processes, etc.), perform purification processes, supplemental amplification processes, and/or perform any suitable processing processes. In an example, the method 100 can include at least one of (e.g., prior to and/or after generating a set of microbial community-associated fragments; etc.): transforming RNA (e.g., microbial RNA) from the sample into cDNA (e.g., for facilitating preparation of a normalized metatranscriptomic-associated library for sequencing ;etc.); performing a first target-capture process (and/or any suitable number of target-capture processes; etc.) to selectively enrich first sequences corresponding to first nucleic acids of the sample; and/or performing a second target-capture process (and/or any suitable number of target-capture processes; etc.) to selectively deplete (e.g., exclude) second sequences corresponding to second nucleic acids of the sample.

Transforming nucleic acids can be used for facilitating detection of expression of target genes and/or other targets (e.g., nucleic acid targets in one or more biological samples), and/or to detect the presence of and/or other suitable characteristics of viruses (e.g., viruses with RNA based genomes, etc.). In an example, processing can include, prior to and/or after fragmentation, transforming mRNA in total nucleic acids into cDNA by reverse transcriptase PCR (RT-PCR) (e.g., where RT-PCR can be performed using random primers, such as to reverse transcribe all or substantially all of the mRNA in a sample; using reverse transcriptase enzyme; poly-T primers; multiple target-specific primers; primers targeting mRNA of interest; and/or other suitable components; etc.),and/or other suitable transformation processes, such as for facilitating fragmentation processes and inclusion in a microbial community-associated library. Performing target-capture processes can include enriching or excluding nucleic acids corresponding to target sequences, and/or enriching or excluding (e.g., depleting) suitable types of targets (e.g., prior to fragmentation processes, etc.), such as where target-capture processes can include oligonucleotide-based processes (e.g., using oligonucleotides immobilized or attached to a bead service, where the oligonucleotides can hybridize with sequences in target nucleic acids such as target DNA fragments, etc.). In variations, enrichment and/or depletion of target nucleic acid products can be performed at any suitable time and frequency, such as in relation to any suitable portions of embodiments of the method 100 (e.g., performed with products from an amplification process for generating a set of sequencing-ready microbial community-associated fragments; etc.). In examples enrichment and/or depletion of target nucleic acid products can be based on targeting hybridization probes (e.g., after fragmentation, after generating normalized microbial community-associated fragments; etc.). In examples, probes can include a string of nucleic acids that can specifically interact with target nucleic acids (e.g., target DNA) through complementary chemical bonds, such as in a sequence-specific manner and that can additionally or alternative include a biotin tag at any position. In specific examples, single stranded DNA and/or RNA probes are designed, such as of 20 to 500 bases in length, and that are complementary to the sequence of the nucleic acid targets. In a specific example, nucleic acids (e.g., at any suitable portion of generating a normalized microbial community-associated library; etc.) are incubated at 60 to 99°C, in a suitable buffer in order to denature and allow separation of complementary strands, where if single stranded nucleic acids (e.g., cDNA) are used, this process can be omitted. In specific examples, probes and nucleic acids of interest are incubated for 10 min and up to 5 days (and/or other suitable time periods), in a temperature of Tm +/-10^{O}C (and/or other suitable temperatures), in which Tm corresponds to the melting temperature of the probe, where this can allow the binding of the probes to the targets of interest, while leaving other non-targeted nucleic acids unbound. In specific examples, the nucleic acid-probe hybrids can then be captured, by specific interaction of the streptavidin protein with the biotin tag in the probes; where streptavidin can be tethered to any type of immobilization surface, including but not restricted to magnetic beads, resins, or surfaces of disposable labware. Additionally or alternatively, ssDNA or ssRNA probes (e.g., of 20 to 500 nucleotides) can be covalently attached to magnetic beads before hybridization, and this complex can be used for incubation with the target nucleic acid; where after capture of the probe-nucleic acid hybrids, the supernatant includes the depleted fraction, and the beads includes the enriched fraction; and where nucleic acids of the fraction of interest can subsequently be recovered.

In a variation, processing can include cleaning the set of microbial community-associated fragments, such as to remove any molecules that interfere with downstream processing, and/or can be size selected, in order to limit the range of fragment size that is desired to be processed. In examples, nucleic acid cleanup and/or size selection can be performed using silica-resin columns, magnetic beads, other types of nucleic acid binding material that allow purification, and/or any other suitable sample processing operations.

In a variation, processing can include repairing microbial community-associated fragments (e.g., fragmented DNA), such as based on (e.g., using, processing with, etc.) one or more enzymes including at least one of: DNA polymerase, 3' exonuclease, polynucleotide kinase, and/or any other suitable enzymes. In examples, enzymes use in repair processes can be removed such as through any suitable purification processes described herein, inactivated (e.g., by incubation at temperatures above 60°C, such as if enzymes used are not thermostable), and/or otherwise processed. In a specific example, microbial community associated fragments (e.g., repaired microbial community-associated fragments) can be 3'-adenylated (e.g., using a DNA polymerase enzyme lacking 3' EXONUCLEASE activity, and dATP, etc.), which can additionally or alternatively be followed by one or more purification processes (e.g., described herein; etc.). However, repair processes can be performed in any suitable manner (e.g., at any suitable time and frequency in relation to any suitable portions of the method 100, etc.).

However, processing the set of total nucleic acids can be performed in addition to and/or alternatively to fragmenting total nucleic acids and/or other suitable components, in addition to and/or alternatively to any suitable portion of generating the set of microbial community-associated fragments; in any suitable portions of embodiments of the method 100 at any suitable time and frequency, and/or in any suitable manner.

However, generating microbial community-associated fragments can be performed in any suitable manner.

### 2.2.B Generating a set of ligated microbial community-associated fragments.

Embodiments of the method 100 can include generating a set of ligated microbial community-associated fragments based on a ligation process S124, which can function to obtain fragments with ligated molecules (e.g., ligated adapter molecules; etc.) for facilitating downstream normalization, other sample processing, and/or bioinformatics analyses.

Generating the set of ligated microbial community associated fragments is preferably based on (e.g., using; process with; perform amplification processes with; etc.) the set of microbial community-associated fragments (e.g., repaired and 3'-adenylated microbial community-associated fragments; microbial community-associated fragments in any suitable processed or non-processed form; etc.), and/or a set of ligation adapter molecules.

Regions (e.g., sequences) added to (e.g., ligated to, such as through a ligation process, etc.) outputs of fragmentation processes (e.g., microbial community-associated fragments of total nucleic acids) preferably include adapter regions (e.g., using ligated adapter molecules; etc.), such as adapter regions enabling binding of primers (e.g., in downstream amplification processes; etc.) and/or other suitable molecules, such as in subsequent portions of embodiments of the method 100 (e.g., subsequent PCR processes; such as in relation to generating sequence-ready microbial community-associated fragments molecules; etc.). However, adding adapter regions and/or other suitable components (e.g., regions of ligation molecules; regions associated with primer regions; etc.) can be performed in any suitable manner.

Ligation adapter molecules (e.g., used in a ligation process for adding to microbial community-associated fragments; etc.) can include any one or more of: external defined sequence regions (e.g., including any characteristics described herein in relation to external defined sequence regions; etc.); adapter regions (e.g., including different adapter region types including distinct sequence elements that facilitate downstream amplification for libraries for NGS and/or other suitable sequencing technologies; sequencing adapter regions associated with the sequencing; etc.); bridge regions (e.g., for tethering both strands of DNA and/or other nucleic acids; etc.); binding regions (e.g., adenine molecules such as for ionic bonding to fragments; phosphate molecules such as for covalent bonding to fragments; and/or other suitable molecules, such as for facilitating any suitable type of binding to microbial community-associated fragments; etc.); and/or any suitable regions. Ligation adapter molecules can be in double-stranded form (e.g., double-stranded DNA ligation adapter molecules; etc.), and/or in any suitable form. In a specific example, generating the set of ligated microbial community-associated fragments can be based on a ligation process with a set of ligation adapter molecules including: first ligation adapter molecules corresponding to a first ligation adapter molecule type of a configuration including "3' EXTERNAL DEFINED SEQUENCE (XXXXXXX)-ADAPTER_ABRIDGE-PHOSPHATE 5'"; and second ligation adapter molecules corresponding to a second ligation adapter molecule type of a configuration including "5' EXTERNAL DEFINED SEQUENCE (YYYYYYY)-ADAPTER_B-BRIDGE-ADENINE 3'"; such as where the "EXTERNAL DEFINED SEQUENCE" can correspond to external defined sequence regions (e.g., including any characteristics described herein in relation to external defined sequence regions; such as of any length and any suitable defined sequence as long as facilitative of subsequent amplification processes; etc.); "ADAPTER_A" and "ADAPTER_B" (and/or any suitable adapter regions of any suitable molecules described herein; etc.) can correspond to two distinct sequence elements that allow downstream amplification for libraries for NGS and/or other sequencing technologies; where "BRIDGE" (and/or other suitable bridge regions of any suitable molecules described herein; etc.) can correspond to a complementary region that tethers both strands of the double-stranded ligation adapter molecule; where "ADENINE" and "PHOSPHATE" molecules (and/or other suitable adenine and/or phosphate molecules of any suitable molecules described herein; etc.) can allow the ionic and covalent bonding respectively microbial community-associated fragments.

In a specific example, ligation adapter molecules can be ligated to both ends of microbial community-associated fragments (e.g., both ends of fragmented DNA; etc.) such as by the addition of ligase enzyme (e.g., DNA ligase enzyme; etc.). In a variation, ligase enzyme and/or other suitable components can be inactivated, such as incubating in temperatures above 6o°C and/or other suitable temperatures. In a variation, purification processes can be performed subsequent to ligation processes (e.g., for DNA purification), and/or at any suitable time and frequency.

In variations, ligation adapter molecules can include one or more UMI regions. For example, the set of ligation adapter molecules can include UMI regions, each UMI region including a set of random "N" bases, where each random "N" base is selected from any one of an "A" base, a "G" base, a "T" base, and a "C" base. In a specific example, ligation adapter molecules (e.g., double stranded DNA ligation adapter molecules' etc.) can correspond to a ligation adapter molecule type of configuration including: "3' EXTERNAL DEFINED SEQUENCE (XXXXXXX)-ADAPTER_ABRIDGE-UNIQUE MOLECULAR IDENTIFIER-PHOSPHATE 5'" and "5' EXTERNAL DEFINED SEQUENCE (YYYYYYY)-ADAPTER_B-BRIDGE-UNIQUE MOLECULAR IDENTIFIER-ADENINE 3'", such as where "UNIQUE MOLECULAR IDENTIFIER" can correspond to UMI regions including a string of "N" deoxynucleotide bases (e.g., "N" being any random base between "A", "C", "T", and "G" nucleic bases, etc.) which can be continuous or separated by defined bases, and which can have a length in between 2 to 20 bases, depending on the amount of molecules and/or variants that are desired to be quantified or differentiated, such as where longer UMI regions allow for a larger number of random base combinations and thus a larger set of unique identifiers. However, ligation adapter molecules including UMI regions can be configured in any suitable manner.

However, ligation adapter molecules can include any suitable regions configured in any suitable manner (e.g., in any suitable order; etc.).

However, generating ligated microbial community-associated fragments can be performed in any suitable manner.

### 2.2.C Generating a set of normalized microbial community-associated fragments.

Embodiments of the method 100 can include generating a set of normalized microbial community-associated fragments (e.g., normalized metagenome-associated fragments; normalized metatranscriptomic-associated fragments; etc.) S126, which can function to obtain normalized Fragments for facilitating preparation of a normalized sequencing library, such as for normalizing representation of different fragments and/or other suitable molecules in the sequencing library. In specific examples, generating a set of normalized microbial community-associated fragments can function to improve equalization of amounts of different types of molecules initially represented in the sample (e.g., improve representation of underrepresented molecules associated with the metagenome and/or metatranscriptome; etc.).

Generating the set of normalized microbial community-associated fragments is preferably based on an amplification process (e.g., PCR process; first amplification process; other suitable types of amplification processes described herein; etc.) with the set of ligated microbial community-associated fragments and/or a set of normalization-based primers (e.g., including any characteristics described herein, etc.), but can be based on any suitable sample processing operations (e.g., ligation operations; purification operations; etc.) and/or any other suitable components.

Normalized microbial community-associated fragments preferably include fragments associated (e.g., attached with; connected to; coupled with; etc.) one or more regions of normalization-based primers, but can additionally or alternatively include any suitable components.

In a specific example, generating the set of microbial community-associated fragments can be based on an amplification process with a set of normalization-based primers (e.g., and a set of ligated microbial community-associated molecules, such as to be used with the amplification process and/or other suitable amplification processes described herein; etc.) including: first primers corresponding to a first primer type configuration including "s'-EXTERNAL DEFINED SEQUENCE (XXXXXXX) -3'", and second primers corresponding to a second primer type configuration including "5'-EXTERNAL DEFINED SEQUENCE (YYYYYYY) -3'"; such as where "EXTERNAL DEFINED SEQUENCE" (and/or other external defined sequence regions of any suitable primers described herein, etc.) can correspond to a defined external sequence (e.g., represented by XXXXXXX in first primer type configuration, and by YYYYYYY in second primer type configuration, etc.), such as sequences of defined external sequence regions of ligated microbial community-associated fragments, such as where the "EXTERNAL DEFINED SEQUENCE" (and/or other external defined sequence regions) can be of any length and any defined sequence. A defined number of molecules of normalization-based primers can be added to the one or more amplification processes for generating a set of normalized microbial community-associated fragments; and/or N number of cycles of PCR (and/or any suitable extent of amplification processes; etc.) can be performed to ensure that all added normalization-based primers are completely used (e.g., with DNA polymerase enzyme, etc.); such as where the number of molecules that can be generated from the one or more amplification processes can be limited to the number of normalization-based primer molecules added (e.g., independent of the number of molecules used as a template for the one or more amplification processes; etc.). In examples, external defined sequence regions of normalization-based primers can anneal to external defined sequence regions of ligated microbial community-associated fragments (e.g., external defined sequence regions added to microbial community-associated fragments, such as based on a ligation process; etc.), such as where the external defined sequence regions of the normalization-based primers can be associated with (e.g., sharing sequences with; complementary to; annealable to; etc.) external defined sequence regions of ligated microbial community-associated fragments. In a specific example, the set of ligation adapter molecules can include first adapter regions associated with the sequencing, where generating the set of ligation adapter molecules can include adding the first adapter regions to the set of microbial community-associated fragments based on the ligation process, where the set of normalized microbial community-associated fragments can include the added first adapter regions, and/or where the set of sequencing-based primers can include second adapter regions for annealing to the added first adapter regions of the set of normalized target molecules.

In variations, generating the set of normalized microbial community-associated fragments (and/or suitable portions of embodiments of the method 100) can include performing one or more purification processes (e.g., to purify any suitable components; to remove any suitable components; etc.). In an example, generating the set of normalized microbial community-associated fragments based on an amplification process (e.g., a PCR process) can include performing a purification process with products of the amplification process to remove the set of normalization-based primers (and/or to remove other suitable components, etc.) from the products of the amplification process. Any suitable purification processes described herein can be used, and/or can be performed in any suitable manner.

Additionally or alternatively, generating normalized microbial community-associated molecules based on one or more amplification processes with normalization-based primers (e.g., for normalizing any suitable molecules) can be performed in any suitable manner analogous to amplification processes described herein with normalization-based primers.

However, generating normalized microbial community-associated fragments can be performed in any suitable manner.

### 2.2.D Generating a set of sequencing-ready microbial community-associated fragments.

Generating a set of sequencing-ready microbial community-associated fragments (e.g., sequencing-ready metagenome-associated fragments; sequencing-ready metatranscriptomic-associated fragments; etc.) S128, which can function to process microbial community-associated fragments (e.g., normalized microbial community-associated fragments) for preparation for sequencing (e.g., NGS, etc.).

Preparing molecules for sequencing preferably includes preparing normalized microbial community-associated fragments for sequencing (e.g., by adding one or more adapter regions and/or one or more index regions, etc.), such as based on an amplification process (e.g., a second amplification process; a second PCR process; etc.), but can additionally or alternatively include preparing any suitable molecules for sequencing.

Generating the set of sequencing-ready microbial community-associated fragments is preferably based on (e.g., use; process with; perform amplification processes with; etc.) a set of normalized microbial community-associated fragments and/or a set of sequencing-based primers (e.g., for incorporation of the sequencing-based primers and/or any suitable regions of sequencing-based primers with the set of normalized microbial community-associated fragments; for adding regions of the sequencing-based primers to the set of normalized microbial community-associated fragments; etc.), but can additionally or alternatively be based on any suitable components. In an example, ligation adapter molecules can include first external adapter regions (e.g., associated with the NGS; etc.), where the set of normalized microbial community-associated fragments (e.g., derived from ligated microbial community-associated fragments generated based on the ligation adapter molecules; etc.) includes the first external adapter regions; and where generating a set of sequencing-ready microbial community-associated fragments (e.g., NGS-ready normalized microbial community-associated fragments; etc.) includes annealing a set of sequencing-based primers (e.g., including an adapter region including external adapter regions, such as complementary external adapter regions to the first external adapter regions, etc.) with the normalized microbial community-associated fragments at the external adapter regions of the normalized microbial community-associated fragments.

Generating sequencing-ready target molecules preferably increases the concentration of normalized microbial community-associated fragments to an amount suitable for NGS and/or other suitable sequencing (e.g., to an amount at or above 2 pM and/or any suitable desired amount; etc.), such as where the sequencing-ready microbial community-associated fragments are at desired amounts (e.g., normalized amounts and suitable amounts for NGS, etc.).

In a specific example, performing a PCR process (e.g., a second PCR process for generating the set of sequencing-ready microbial community-associated fragments) can include using between and/or including 5-45 cycles of PCR (and/or any suitable number of cycles of PCR).

In a specific example, generating the set of sequencing-ready microbial community-associated fragments can be based on an amplification process with a set of sequencing-based primers (e.g., and a set of normalized microbial community-associated fragments, such as to be used with the amplification process and/or other suitable amplification processes described herein; etc.) including: first sequencing-based primers corresponding to a first primer type configuration including "5'-SEQUENCING ADAPTER-SEQUENCING INDEX-ADAPTER_A-3'", and second sequencing-based primers corresponding to a second primer type configuration including "5'-SEQUENCING ADAPTER-SEQUENCING INDEX-ADAPTER_B-3'"; such as where "SEQUENCING INDEX" (and/or other index regions of any suitable primers described herein, etc.) can correspond to a defined barcode sequence of any suitable length) that can allow for combinatorial tagging of different samples that are going to be sequenced; such as where "SEQUENCING ADAPTER" (and/or other adapter regions of any suitable primers described herein, etc.) can correspond to any specific sequence that is required by and/or otherwise associated with one or more NGS technologies and/or other suitable sequencing technologies, such as for enabling sequencing to occur; such as where "ADAPTER_A" and "ADAPTER_B" can include any suitable characteristics of "ADAPTER_A" and/or "ADAPTER_B" described herein and/or of any suitable adapter regions.

In variations, generating the set of sequencing-ready microbial community-associated fragments (and/or suitable portions of embodiments the method 100) can additionally or alternatively include performing one or more supplementary amplification processes (e.g., described herein, etc.) and/or other suitable processes for increasing concentration of molecules for a sequencing library (e.g., described herein, etc.).

In a specific example, sequencing-ready microbial community-associated fragments (and/or suitable products of a normalized amplicon library) can include a configuration including: "5'-SEQUENCING ADAPTER- SEQUENCING INDEX-EXTERNAL ADAPTER-TARGET SEQUENCE-EXTERNAL ADAPTER-SEQUENCING INDEX-SEQUENCING ADAPTER-3'". In a specific example, such as where ligation adapter molecules including UMI regions are used (e.g., in generating ligated microbial community-associated fragments, etc.), sequencing-ready microbial community-associated fragments (and/or suitable products of a normalized amplicon library) can include a configuration including "5'-SEQUENCING ADAPTER-SEQUENCING INDEX- EXTERNAL ADAPTER-UNIQUE MOLECULAR IDENTIFIER-TARGET_SEQUENCE-UNIQUE_MOLECULAR_IDENTIFIER-EXTERNAL ADAPTER-SEQUENCING INDEX-SEQUENCING ADAPTER-3'".

Additionally or alternatively, generating sequencing-ready microbial community-associated fragments based on one or more amplification processes with sequencing-based primers (e.g., including any suitable characteristics described herein in relation to sequencing-based primers; etc.) can be performed in any suitable manner analogous to amplification processes described herein with sequencing-based primers.

However, generating the set of sequencing-ready microbial community-associated fragments can be performed in any suitable manner.

### 2.3 Generating a Combined Sequencing Library.

As shown in FIG. 1-2, embodiments of the method 100 can include generating a combined library associated with amplicon-associated sequencing and microbial community-associated sequencing (e.g., metagenomic-associated sequencing and/or metatranscriptomic-associated sequencing; etc.) S150, which can function to facilitate combined sequencing associated with both amplicon-associated sequencing and microbial community-associated sequencing. In an example, portions of embodiments of the method 100 can include identifying specific microorganisms (and/or performing suitable microbiome characterization in relation to microbiome composition, function, and/or suitable microorganism-related aspects) (e.g., determining abundance of, presence of, absence of, one or more microorganism taxa, etc.) from the microbial community based on a microorganism sequence dataset derived from sequencing of the normalized combined sequencing library.

Additionally or alternatively, generating a combined library S150 can include generating a normalized mixture based on performing a normalization process (e.g., a second amplification process, such as a second PCR process, such as where a first amplification process can be used for generating amplicon target molecules; etc.) S156, such as with the set of amplicon target molecules and the set of microbial community-associated fragments; generating a normalized combined library (e.g., including a set of sequencing-ready target molecules, etc.) based on the normalized mixture (e.g., based on a third amplification process with the normalized mixture; based on a third PCR process with the normalized mixture and the set of sequencing-based primers; etc.) S158; and/or other suitable processes.

Additionally or alternatively, generating a combined sequencing library S150 can include any suitable portions of embodiments of the method 100 including generating a normalized amplicon library S110 (e.g., generating a set of amplicon target molecules S112; etc.), generating a normalized microbial community-associated library S120 (e.g., generating a set of microbial community-associated fragments S122; generating a set of ligated microbial community-associated fragments S124; etc.), and/or other suitable portions of embodiments of the method 100, such as in relation to facilitating generation of a normalized combined sequencing library.

Combined sequence libraries preferably include components (e.g., sequencable components, normalized molecules, target molecules, fragments of total nucleic acids, amplicon-associated components, microbial community-associated components, etc.) associated with amplicon-associated sequencing (e.g., components including amplicons; processed amplicons, such as for preparation for sequencing, such as processed in relation to microbial community-associated components, such as processed in relation to balancing concentration ratios between amplicon-associated components and microbial community-associated components; outputs associated with amplicon generation and/or processing; etc.) and microbial community-associated sequencing (components including fragments of total nucleic acids; processed fragments, such as for facilitating sequencing; total nucleic acids themselves; etc.), but can additionally or alternatively include any suitable components. Combined sequencing libraries (e.g., normalized combined sequencing libraries; etc.) preferably include a set of sequencing-ready molecules associated with the set of targets (e.g., associated with amplicon-associated sequencing, etc.) and the microbial community (e.g., associated with microbial community-associated sequencing; etc.).

Amplicon-associated sequencing preferably includes sequencing associated with analysis of a single or small number of targets (e.g., gene regions), such as for identification of one or more microorganism taxa in a biological sample, but can additionally or alternatively include any suitable sequencing associated with amplicons. Microbial community-associated sequencing preferably includes sequencing associated with analysis of a microbial community and/or other suitable ecological communities (e.g., present in one or more biological samples), such as including a whole community of DNA (e.g., and/or RNA transcribed into cDNA; etc.) as opposed to analysis of a single gene amplicon, but can additionally or alternatively include any suitable sequencing associated with microbial communities (e.g., in relation to composition-related analysis; function-related analysis; etc.), ecological communities, groups of microorganisms, metatranscriptomic-related aspects, and/or metagenome-related aspects.

Combined sequencing libraries can additionally or alternatively include one or more aggregate sequencing libraries (separate mixtures; separate sub-libraries, such as for use in different compartments in multiplex sequencing; etc.).

Portions of preparing a combined sequencing library can be performed with any suitable relationship (e.g., temporal relationship, such as before, after, during, serially, in parallel; relationships regarding components used as inputs and/or generated as outputs; etc.) with portions of embodiments of the method 100.

In variations, portions of preparing one or more combined sequencing libraries can include any suitable processes (and/or analogous processes) described in relation to any suitable portions of generating one or more normalized amplicon libraries S110, generating one or more normalized microbial community-associated libraries S120, and/or other suitable portions of embodiments of the method 100. However, preparing a combined sequencing library S150 can be performed in any suitable manner.

### 2.3.A Generating a Normalized Mixture.

Embodiments of the method can include generating a normalized mixture based on performing a normalization process (e.g., a second amplification process, such as a second PCR process; etc.) with the set of amplicon target molecules and the set of microbial community-associated fragments S156, which can function to combine (e.g., aggregate) products associated with preceding processing processes (e.g., amplicon target molecules, microbial community-associated fragments such as ligated microbial community-associated fragments, etc.) for facilitating amplicon-associated sequencing and microbial community-associated sequencing.

Generating a normalized mixture is preferably based on (e.g., includes) performing one or more normalization processes, which preferably includes an amplification process (e.g., a second amplification process including a second PCR process, such as where generating the amplicon target molecules includes a first amplification process including a first PCR process; an amplification process including balancing of concentration ratios between amplicon target molecules, microbial community-associated fragments, and/or other suitable components; etc.), but normalization processes can include any suitable processes (e.g., pre-processing processes; etc.) for facilitating generation of one or more normalized mixtures. In an example, performing a normalization process can include performing a PCR process (e.g., a second PCR process) with a set of amplicon target molecules, a set of ligated microbial community-associated fragments, and a set of normalization-based primers (e.g., where the set of normalization-based primers includes primers associated with, such as complementary to components of, the set of amplicon target molecules and the set of microbial community-associated fragments; etc.). In an example, the set of normalization-based primers (and/or sequencing-based primers and/or other suitable primers usable in generating a combined sequencing library; etc.) can include a set of amplicon-associated primers associated with (e.g., including sequence regions complementary to sequence regions of, etc.) amplicon target molecules; and a set of microbial community-associated primers associated with (e.g., including sequence regions complementary to sequence regions of, etc.) microbial community-associated fragments (e.g., ligated microbial community-associated fragments; etc.). In a specific example, the set of amplicon-associated primers can include a first subset of amplicon-associated primers, where each amplicon-associated primer of the first subset includes a first adapter region associated with the NGS and the first subset of amplicon-generation primers (e.g., where the first adapter region is complementary to, bindable to, coupleable to, and/or otherwise associated with amplicon-generation adapter regions of the first subset of amplicon-generation primers, etc.); and a second subset of amplicon-associated primers, where each amplicon-associated primer of the second subset includes a second adapter region associated with the NGS and the second subset of amplicon-generation primers (e.g., where the second adapter region is complementary to, bindable to, coupleable to, and/or otherwise associated with amplicon-generation adapter regions of the second subset of amplicon-generation primers, etc.), and where microbial community-associated primers of the set of microbial community-associated primers can include microbial community-associated adapter regions associated with the NGS and added adapters of the set of ligated microbial community-associated fragments (e.g., where the microbial community-associated adapter regions are complementary to, bindable to, coupleable to, and/or otherwise associated with added adapter regions, such as added adapters, of the set of ligated microbial community-associated fragments; etc.). In a specific example, the set of normalization-based primers can include primers including configurations including "s'-EXTERNAL ADAPTER-ADAPTER A1-3'" + "5'-EXTERNAL ADAPTER-ADAPTER A2-3'" (e.g., a first type of primer pair; etc.) and/or "5'-EXTERNAL ADAPTER-ADAPTER M1-3'" + "s'-EXTERNAL ADAPTER-ADAPTER M2-3'" (e.g., a second type of primer pair; etc.), such as where "ADAPTER-A1" and "ADAPTER-A2" regions can enable amplification of amplicon target molecules (e.g., amplification of amplicon DNA, etc.), and "ADAPTER-M1" and "ADAPTER-M2" can enable amplification of microbial community-associated fragments (e.g., metagenome DNA; metagenomic-associated fragments; metatranscriptomic-associated fragments; etc.), and/or the set of normalization-based primers can include primers including any suitable configurations. In a specific example, generating the normalized mixture can be based on a second amplification process with the set of amplicon target molecules (e.g., where generating the amplicon target can be based on a first amplification process; etc.), the set of microbial community-associated fragments, and a set of normalization-based primers including external defined sequence regions for annealing to the set of amplicon target molecules and the set of microbial community-associated fragments. However, amplicon-associated primers, microbial community-associated primers, and/or other suitable primers usable in generating a combined sequencing library can be configured in any suitable manner.

Generating a normalized mixture includes modifying a concentration ratio between amplicon target molecules and microbial community-associated fragments, and/or between any suitable components. Modifying a concentration ratio can include adding, in limiting amounts, normalization-based primers (e.g., pairs of normalization-based primers, such as first pairs of amplicon-associated primers, and second pairs of microbial community-associated primers; such as primer pairs including configurations described herein, etc.), for adjusting the concentration ratio to a desired concentration ratio (e.g., in relation to reads associated with amplicon target molecules and reads associated with microbial community-associated fragments, etc.). In a variation, modifying a concentration ratio can include modifying the concentration ratio independent of a PCR process. In an example, modifying the concentration ratio can include cleaning samples (e.g., samples including the amplicon target molecules; samples including the microbial community-associated fragments; mixture samples including both the amplicon target molecules and the microbial community-associated fragments; etc.); measuring associated nucleic acids (e.g., associated DNA, etc.) with fluorometric, spectrophotometric, and/or other suitable concentration measuring processes; and modifying the concentration ratio based on a desired ratio of amplicon-associated reads and microbial community-associated sequencing reads. In an example, generating the normalized mixture includes performing the normalization process based on adjustment of a concentration ratio between the set of amplicon target molecules and the set of microbial community-associated fragments, and where a set of sequencing-based primers (e.g., for generating a set of sequencing-ready target molecules, etc.) can include adapter regions associated with the NGS, amplicon-associated adapter regions of the set of amplicon-generation primers, and added adapter regions (e.g., added adapters; microbial community-associated fragment-generation primers; etc.) of the set of microbial community-associated fragments. In a specific example, generating the normalized mixture includes adjusting a concentration ratio between the set of amplicon target molecules and the set of microbial community-associated fragments (e.g., ligated microbial community-associated fragments; etc.), based on a desired ratio of amplicon-associated sequencing reads and microbial community-associated sequencing reads. Additionally or alternatively, modifying concentration ratios can be performed in any suitable manner.

However, generating one or more normalized mixtures can be performed in any suitable manner.

### 2.3.C Generating a Normalized Combined Library.

Embodiments of the method 100 can include generating a normalized combined library including a set of sequencing-ready (e.g., NGS-ready) molecules (e.g., sequencing-ready target molecules; sequencing-ready microbial community-associated fragments; etc.) S158, which can function to process one or more amplicon target molecules and/or microbial community-associated fragments (e.g., ligated microbial community-associated fragments; etc.), and/or other suitable mixtures (e.g., of amplicon-associated components and microbial community-associated components, etc.) for preparation for sequencing (e.g., NGS; sequencing including simultaneous amplicon-associated sequencing and microbial community-associated sequencing; etc.). In an example, generating a normalized combined library including sequencing-ready molecules can be based on a set of amplicon target molecules, a set of microbial community-associated fragments (e.g., ligated microbial community-associated fragments; etc.), and a set of sequencing-based primers.

Sequencing-ready molecules are preferably associated with the one or more targets (e.g., associated with the amplicons) and the microbial community (e.g., associated with the microbial community-associated fragments; where targets can include total nucleic acids; where targets can be associated with a plurality of taxa of microorganisms; etc.), but can additionally or alternatively be associated with the one or more targets independent of the microbial community; the microbial community independent of the one or more targets; and/or any other suitable targets of interest. Generating sequencing-ready molecules is preferably based on (e.g., includes) an amplification process (e.g., a third amplification process including a third PCR process, where generating the amplicon target molecules can include a first amplification process including a first PCR process; where generating the normalized mixture can include a second amplification process including a second PCR process; etc.), such as with the set of amplicon target molecules, the set of microbial community-associated fragments, and the set of sequencing-based primers. The PCR process preferably includes limited cycles (e.g., fewer than a threshold amount, etc.), but can include any suitable number of cycles, etc.). Performing the amplification process (e.g., for generating sequencing-ready molecules; etc.) preferably includes adding one or more adapter regions and/or one or more index regions (e.g., through the amplification process) to the components (e.g., of the mixture) such as including the amplicon target molecules and/or microbial community-associated fragments, but adapter regions, index regions, and/or other suitable regions (e.g., primer regions described herein, etc.) can be added in any suitable manner (e.g., ligation processes, etc.). In an example, sequencing-based primers can include index regions (e.g., including sequencing index regions, etc.) configured to facilitate multiplexing associated with the sequencing (e.g., NGS, etc.), and adapter regions associated with the sequencing (e.g., NGS, etc.) and one or more primers and/or adapter regions (e.g., primers used in generating amplicon target molecules such as adapter regions of the primers; adapter regions of amplicon target molecules; adapter regions of microbial community-associated fragments; where sequencing-based primers can include adapter regions complementary, annealable to, and/or otherwise associated with the adapter regions of amplicon target molecules and/or adapter regions of microbial community-associated fragments; and/or other suitable components; etc.). In a variation, sequencing-based primers can include regions (e.g., adapter regions, etc.) configured to anneal with adapter regions (e.g., amplicon-associated adapter regions; microbial community-associated adapter regions; etc.) of amplicon target molecules and/or microbial community-associated fragments, and/or other suitable components (e.g., included in a mixture including the amplicon target molecules and ligated microbial community-associated fragments, etc.). In an example, sequencing-based primers can include regions configured to anneal with amplicon-generation primer adapter regions and/or other suitable adapter regions (e.g., microbial community-associated adapter regions of microbial community-associated fragments, etc.). In an example, generating the normalized combined library includes generating the normalized combined library based on the third amplification process with the normalized mixture and a set of sequencing-based primers including adapter regions associated with the sequencing. Additionally or alternatively, sequencing-based primers associated with S158 can be the same, similar to, different, and/or include any suitable characteristics described herein in relation to sequencing-based primers. However, sequencing-based primers can be configured in any suitable manner, and performing amplification processes (e.g., PCR processes) in relation to generating sequencing-ready molecules can be performed in any suitable manner.

In variations, generating sequencing-ready molecules can include performing one or more pre-processing processes and/or post-processing processes. In an example, generating sequencing-ready molecules can include performing a PCR process with the amplicon target molecules, the microbial community-associated fragments, and the set of sequencing-based primers; and cleaning, size-selecting, performing supplementary amplification processes, purifying, enriching, excluding, and/or performing any suitable processes with the products of the amplification process (e.g., for preparing sequencing-ready target molecules suitable for any suitable sequencing technologies; etc.).

However, generating a normalized combined library including sequencing-ready molecules can be performed in any suitable manner.

### 2.4 Magnetic-based normalization process.

Embodiments of the method 100 can additionally or alternatively include performing one or more magnetic-based normalization processes S160, which can function to perform an additional or alternative normalization process to supplement and/or substitute any suitable portions of embodiments of the method 100. The magnetic-based normalization processes can additionally or alternatively function to directly pull magnetic particles of interest from any suitable solutions (e.g., described herein in relation to any suitable portions of embodiments of the method 100; etc.) by the use of a magnetic field that can make the process of magnetic particle handling independent of the use of tips and changing a pipetting head tool. The magnetic-based normalization processes can additionally or alternatively function to facilitate normalization (e.g., by reducing dispersion amongst components of a sample; for equilibrating the number of reads generated during sequencing, etc.); reduce turnaround time and/or costs; improve automation and/or scalability to multiple plates; etc.)

Performing magnetic-based normalization processes can include any one or more of: diluting magnetic beats (e.g., to a desired ratio; etc.); generating a set of bead-molecule compounds (e.g., compounds including a magnetic beads bound to one or more molecules such as target molecules, fragments, etc.), such as based on a binding process with the magnetic beads and suitable molecules; attaching the set of bead-molecule compounds to an interface plate of a magnetic field system; separating the set of bead-molecule compounds from the interface plate; and/or other suitable processes associated with magnetic-based normalization. In an example (e.g., associated with generating a normalized combined library; etc.), performing an additional normalization process on the set of sequencing-ready molecules can include: diluting magnetic beads to a desired ratio; generating a set of bead-molecule compounds based on a binding process with the magnetic beads and the set of sequencing-ready molecules; attaching the set of bead-molecule compounds to an interface plate of a magnetic field system; and/or separating the set of bead-molecule compounds from the interface plate. In an example (e.g., associated with generating a normalized amplicon library; etc.), performing an additional normalization process on the set of sequencing-ready target molecules can include: diluting magnetic beads to a desired ratio; generating a set of bead-target molecules based on a binding process with the magnetic beads and the set of sequencing-ready target molecules; attaching the set of bead-target molecules to an interface plate of a magnetic field system; and separating the set of bead-target molecules from the interface plate. In an example (e.g., associated with generating a normalized microbial community-associated library; etc.), performing an additional normalization process on the set of sequencing-ready microbial community-associated fragments can include: diluting magnetic beads to a desired ratio; generating a set of bead-fragments based on a binding process with the magnetic beads and the set of sequencing-ready microbial community-associated fragments; and attaching the set of bead-fragments to an interface plate of a magnetic field system; and separating the set of bead-fragments from the interface plate.

In a specific example,, magnetic beads are diluted in an appropriate buffer (e.g., polyethylene glycol 20% 2M NaCl) to a desired ratio (e.g., 1:10, 1:20, 1:40, other suitable ratios; etc.); where by diluting the beads, the number of available sites for nucleic acids to bind is limited in a controlled way, allowing to decrease and normalize the number of molecules between samples; where in samples with a higher number of nucleic acid molecules than binding sites on the surface of the beads, the unbound portion can be removed during additional or alternative washing steps; where the diluted beads can be added to the sample (e.g. nucleic acids, PCR amplicons, target molecules; microbial community-associated fragments, etc.) and allowed to incubate for a variable time; where a magnetic tool covered by a plate (e.g., new 96-well polystyrene plate; etc.) in the bottom (e.g., where the plate can act as a clean interface between a magnetic field system and samples) can be submerged into a plate including samples and beads; where after an incubation, beads can attach to the surface of the interface plate, which can be lifted to remove magnetic beads and attached nucleic acids and/or other suitable molecules from the rest of the sample; where the attached beads can be submerged twice in new plates including a washing solution (e.g. ethanol 80%), further cleaning the nucleic acid molecules; where beads can then be allowed to dry for a few minutes; and/or where beads are submerged in water or any suitable solution to release the nucleic acids, which can be directly used in any suitable portions of embodiments of the method (e.g., for several downstream applications, such as PCR, RT-PCR, library preparation, restriction enzyme analysis, ligation, sequencing, etc.).

Performing magnetic-based normalization processes can be performed at any suitable time and frequency, using any suitable inputs (e.g., any suitable inputs into or products of any suitable amplification processes and/or other suitable sample processing operations; etc.).

However, performing one or more magnetic-based normalization processes can be performed in any suitable manner.

### 3. Examples.

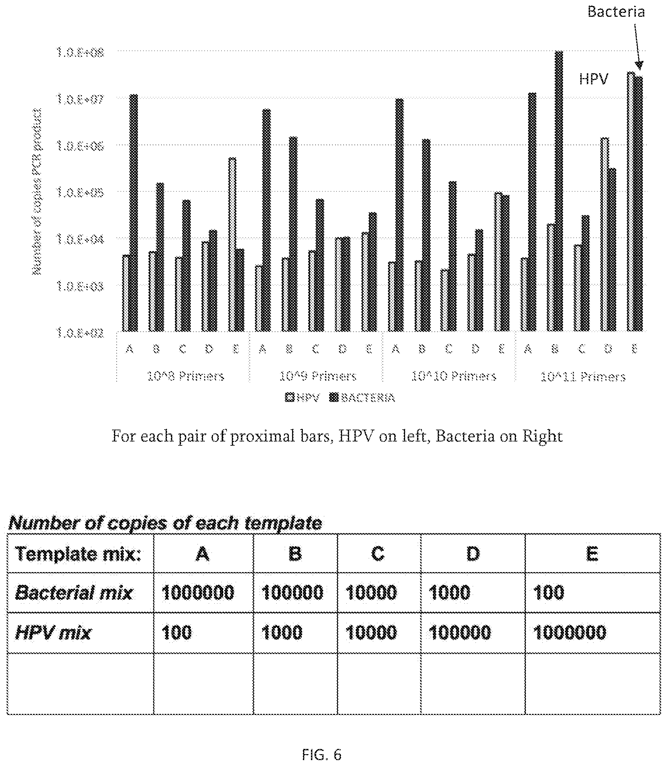

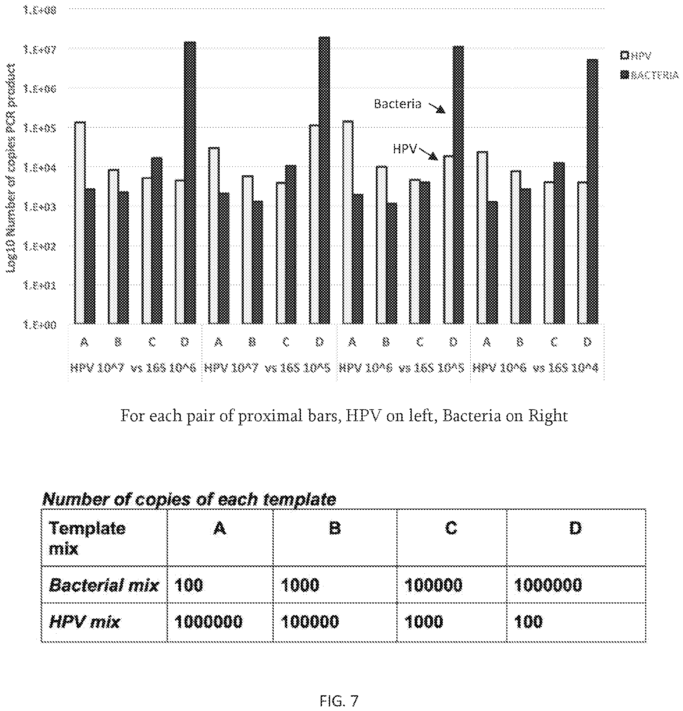

In an example of normalized amplicon library generation: balanced NGS libraries including variable amounts of bacterial DNA and HPV viral DNA were constructed, and then concentration of each target measured by qPCR; the library was tested with wide variations in target DNA concentration in every mix, and equal amounts of normalization-based primers; where as shown in FIG. 6, when using equal amounts of normalization-based primers, with concentrations below 10¹⁰ primer copies/reaction, allow to completely or partially normalize both bacterial and HPV target copy numbers that differ in a ratio of 1:100 in initial input (template mixes B and D) (e.g., as shown in FIG. 6 showing quantitation by qPCR, where a same concentration of HPV and 16S primers were used in each reaction, and also different concentrations of the conditions were tested ( 10⁸, 10⁹, 10¹⁰ and 10¹¹ molecules of normalization-based primers); where the template included synthetic template of HPV 18+31, and a gDNA bacterial mix (*P*. *aeuruginosa, S. aureus and E.faecalis*) was used at different number of copies (A, B, C, D and E). In a similar example, using normalization-based primers in a 1:10 ratio to better normalize the amplicon copy numbers for HPV: as shown in FIG. 7, mixes containing 1:100 difference in initial input are completely normalized (mixes B and C), and samples containing 1:10000 difference in initial input are partially normalized (mixes A and D); where FIG. 7 shows quantitation by qPCR, showing different concentration of HPV and 16S primers tested, where HPV primers have been added in a higher concentration vs. the 16S ones; and a difference between one order or two orders in the number of molecules of primers was tested; where the different concentrations for HPV/16S molecules tested were: 10^7/10^6, 10^7/10^5, 10^6/10^5 and 10^6/10^4; and with template including synthetic template of HPV 18+31, and a gDNA bacterial mix (P. *aeuruginosa, S. aureus and E.faecalis*) was used at different number of copies (A, B, C and D).

In an example, portions of embodiments of the method 100 can include preparing combined sequencing libraries from human stool biological samples, but combined sequence libraries can additionally or alternately be prepared from any suitable biological samples (e.g., from any suitable users; from any suitable collection sites; etc.). In specific examples, a combined sequence library can be constructed from a stool sample from a single user; bacterial taxa analysis of samples from a plurality (e.g., hundreds, etc.) of sequencing runs can show statistically significant reproducible diversity (e.g., indicating robustness and consistency; etc.). In a specific example, a combined sequencing library can lead to results illustrating inclusion of all species (and/or other suitable taxa) represented in amplicon-associated components of the combined sequencing libraries, and higher representation of bacterial taxa shown to be underrepresented when using only amplicon-focused approaches (e.g., Tenericutes phylum, etc.). In specific examples, processes associated with preparing a combined sequencing library can be used for identification of different organisms and specific nucleic acid targets of interest, by using amplicon-associated processes to identify the presence or absence of a given microorganism (e.g., including and/or based on 16S regions, 18S regions, ITS, etc.), and by using microbial community-associated processes to identify the nucleic acid target of interest (e.g., antibiotic resistance genes, virulence factors, secretion systems, etc.) and/or other suitable targets

In examples, the method 100 and/or system 200 can confer improvements over conventional approaches. Specific examples of the method 100 and/or system 200 can confer technologically-rooted solutions to at least the challenges associated with conventional approaches. In examples, the technology can transform entities (e.g., biological samples, targets such as nucleic acid targets, primers, users, etc.) into different states or things. In a specific example, target molecules can be transformed into sequencing-ready target molecules, and/or microbial community-associated nucleic acids can be transformed into sequencing-ready microbial community-associated fragments, such as adapted for improved sequencing (e.g., associated with reduced biases, improved analyses such as improved quantification, etc.). In a specific example, improved sequencing libraries can be prepared, leading to improved microbiome characterizations, such as for facilitating improved diagnosis and/or therapy associated with one or more microorganism-related conditions, thereby transforming one or more users. However, in examples, the technology can transform entities in any suitable manner.

In examples, the technology can improve technical fields of at least sequencing library preparation, sample processing, genomics, molecular biology, microbiology, diagnostics, therapeutics, digital medicine, modeling, and/or other suitable technical fields. However, in specific examples, the technology can provide any other suitable improvements, such as by performing portions of embodiments of the method 100 and/or system 200.

### 3.A Magnetic-based normalization examples.

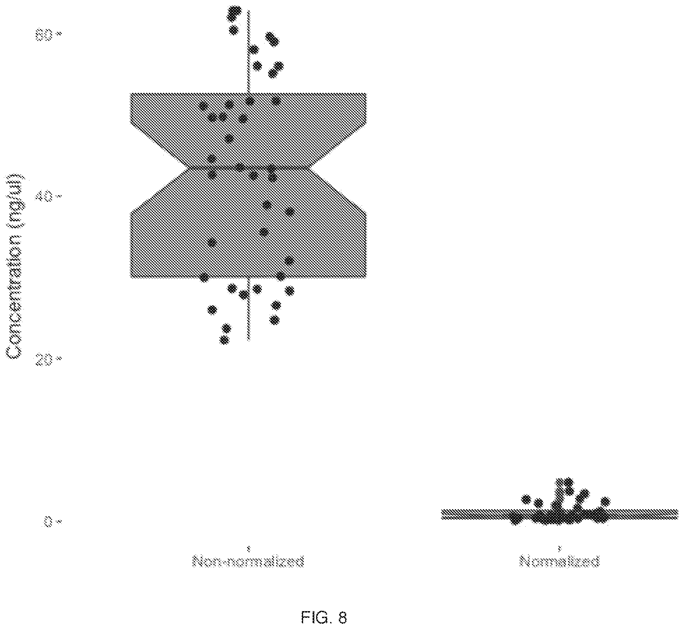

In an example, magnetic-based normalization can be performed for 16S gene normalization after PCR: DNA extracted from mouth and gut samples was used as a template to amplify the 16S gene with a 30-cycles PCR; after PCR, 3 ul of each reaction were taken and the amount of DNA was normalized among samples through the use of magnetic beads. Reactions before and after normalization were quantified (e.g., as shown in FIG. 8 including a box plot comparing the concentration of 16S PCRs before and after normalization, where each dot represents an individual sample, and boxes show the median and the 95% confidence interval; etc.); where normalized samples had a lower concentration than non-normalized samples, with the average decreasing from 43.18 ng/ul to 1.11 ng/ul; where this indicates that the bead normalization can effectively reduce DNA concentration; and where the distribution of the samples decreased considerably after normalization

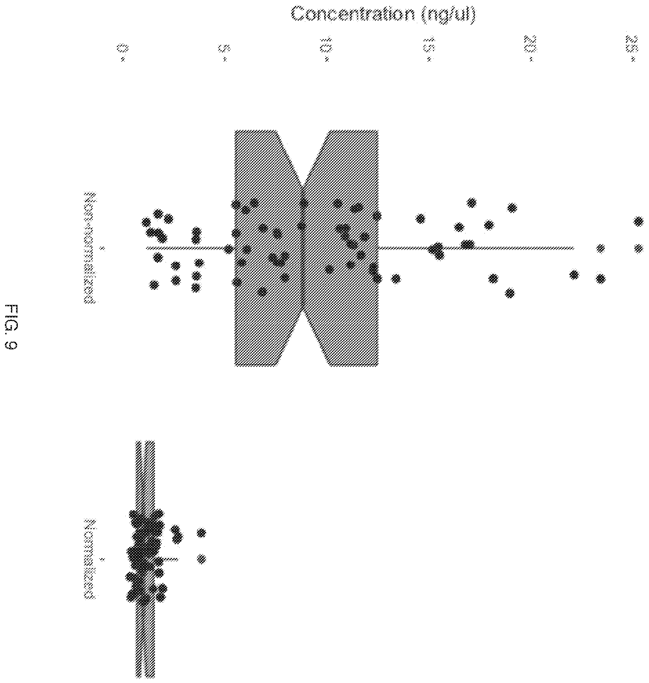

In an example, magnetic-based normalization can be performed in relation to ITS gene normalization after PCR: DNA extracted from gut and genital samples was used as a template to amplify the ITS gene; PCR products were quantified with before and after bead normalization; revealing that the normalization decreased DNA concentration and also balanced concentration among samples (e.g., as shown in FIG. 9 including a box plot comparing the concentration of ITS PCRs before and after normalization, where each dot represents an individual reaction, and boxes show the median and the 95% confidence interval; etc.), such as with the average concentration decreasing from 10.29 ng/ul to 1.19 ng/ul; and where the distribution of the samples also decreased considerably after normalization.

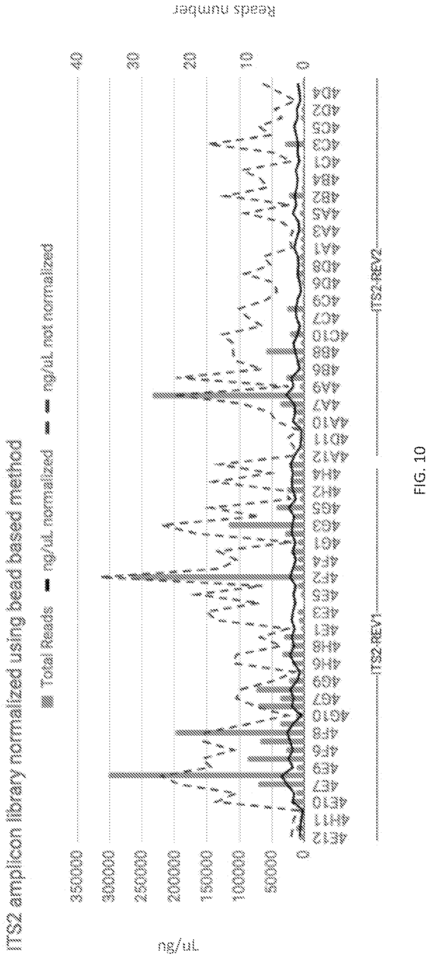

In an example of effects of normalization in a sequencing run: equal amounts of ITS PCRs were consolidated into one library, quantified by qPCR and loaded in the sequencer; where, FIG. 10 can include the number of total reads per sample and a comparison to the DNA concentration before and after normalization (e.g., where certain lines show the concentration (ng/ul DNA) for each sample before normalization, certain lines show the concentration after normalization, and bars show total reads for each sample., where samples are ordered according to the ITS primers used for PCR; etc.).

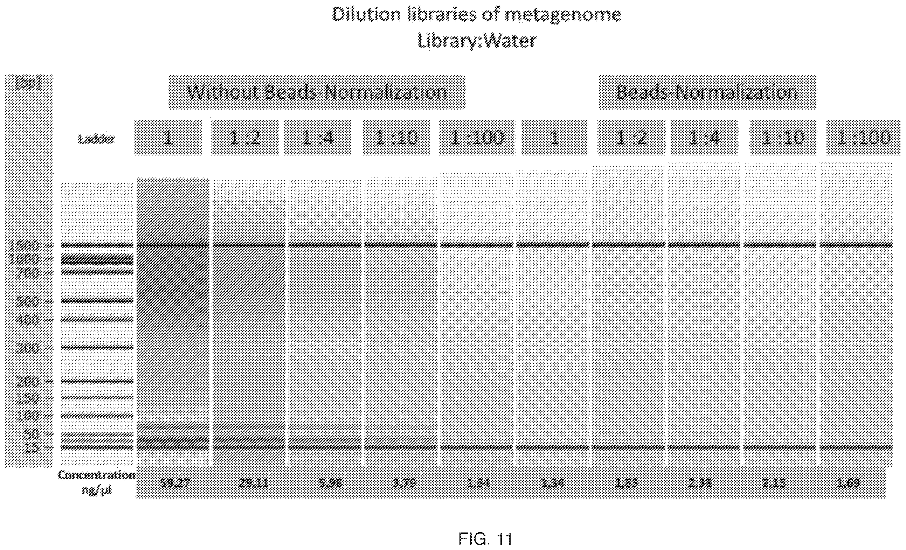

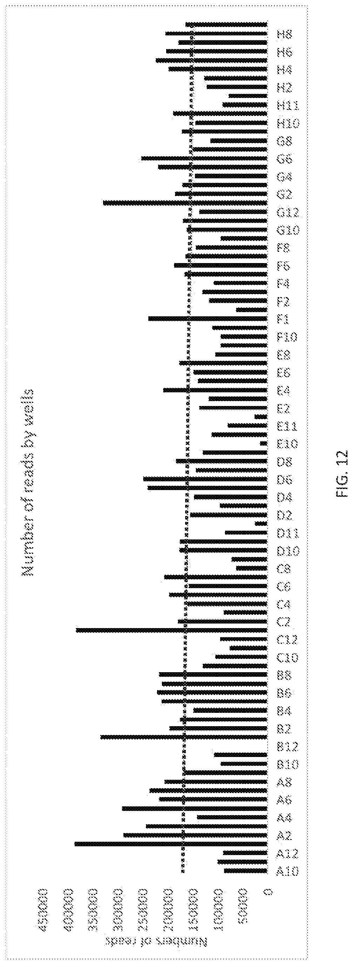

In an example of normalization of a microbial community-associated library (e.g., metagenomic library, etc.): serial dilutions were made (undiluted, 1:2, 1:4, 1:10, 1:100) from a previously non-normalized and bead-normalized metagenome library using 1:40 ratio of beads:PEG and 5 ul of DNA; the library was quantified and analyzed by (e.g., as shown in FIG. 11 including visualization and quantification of a diluted metagenomic library (1:2, 1:4, 1:10 and 1:100) with or without magnetic-based normalization (e.g., bead-based normalization); etc.); where results indicate normalization of the concentration libraries using a magnetic bead-based normalization process on developed metagenomic library construction. In the example, for a metagenomic seq run using bead-based normalized samples: the normalized and non-normalized libraries were tested by sequencing run, with the aim to see if our new bead based method can normalize the number of read per sample (e.g., mouth samples, gut samples; etc.); where samples were normalized using 1:40 of bead:PEG ratio, consolidated and loading in the Miseq sequencer; where FIG. 12 includes number of total reads per sample and the line shows the average of reads from sequenced samples; where results indicate a homogenous number of reads with an average of 157.728.9 reads per sample (e.g., as shown in FIG. 11-12).

### 4. Other

Embodiments of the method 100 can, however, include any other suitable blocks or steps configured to facilitate reception of biological samples from subjects, processing of biological samples from subjects, analyzing data derived from biological samples, and generating models that can be used to provide customized diagnostics and/or probiotic-based therapeutics according to specific microbiome compositions and/or functional features of subjects.

Embodiments of the method 100 and/or system 200 can include every combination and permutation of the various system components and the various method processes, including any variants (e.g., embodiments, variations, examples, specific examples, figures, etc.), where portions of embodiments of the method 100 and/or processes described herein can be performed asynchronously (e.g., sequentially), concurrently (e.g., in parallel), or in any other suitable order by and/or using one or more instances, elements, components of, and/or other aspects of the system 200 and/or other entities described herein.

Any of the variants described herein (e.g., embodiments, variations, examples, specific examples, figures, etc.) and/or any portion of the variants described herein can be additionally or alternatively combined, aggregated, excluded, used, performed serially, performed in parallel, and/or otherwise applied.

Portions of embodiments of the method 100 and/or system 200 can be embodied and/or implemented at least in part as a machine configured to receive a computer-readable medium storing computer-readable instructions. The instructions can be executed by computer-executable components that can be integrated with the system. The computer-readable medium can be stored on any suitable computer-readable media such as RAMs, ROMs, flash memory, EEPROMs, optical devices (CD or DVD), hard drives, floppy drives, or any suitable device. The computer-executable component can be a general or application specific processor, but any suitable dedicated hardware or hardware/firmware combination device can alternatively or additionally execute the instructions.

As a person skilled in the art will recognize from the previous detailed description and from the figures and claims, modifications and changes can be made to embodiments of the method 100, system 200, and/or variants without departing from the scope defined in the claims.

## Claims

1. A method for preparation of a normalized combined library, the method comprising:
generating a set of amplicon target molecules based on a first polymerase chain reaction (PCR) process with target molecules of at least one sample including microorganisms from the microbial community, wherein the target molecules correspond to the set of targets associated with one or more regions of amplicon-generation primers, wherein the set of amplicon target molecules comprises external defined sequence regions and adapter regions;
generating a set of microbial community-associated fragments based on processing total nucleic acids from the at least one sample, wherein the set of microbial community-associated fragments comprises at least one of a set of microbial DNA fragments and a set of microbial RNA fragments, and generating a set of ligated microbial community-associated fragments based on a ligation process with the set of microbial community-associated fragments and a set of ligation adapter molecules, wherein the set of ligation adapter molecules comprise external defined sequence regions and adapter regions associated with the sequencing, and wherein the set of ligated microbial community-associated fragments comprises external defined sequence regions and adapter regions;
generating a normalized mixture based on a second PCR process with the set of amplicon target molecules, the set of microbial community-associated fragments and a set of normalization-based primers, wherein the set of normalization-based primers includes a set of amplicon-associated primers associated with amplicon target molecules and a set of microbial community-associated primers associated with microbial community-associated fragments, and wherein the set of normalization-based primers comprise external defined sequence regions for annealing to the set of amplicon target molecules and the set of microbial community-associated fragments, wherein a defined number of molecules of normalization-based primers is added to ensure that all added normalization-based primers are completely used; and
generating the normalized combined library comprising a set of sequencing-ready molecules based on a third PCR process with the normalized mixture and a set of sequencing-based primers comprising adapter regions associated with the sequencing, wherein the set of sequencing-ready molecules is associated with the set of targets and the microbial community-associated fragments, wherein the set of sequencing-based primers comprise adaptor regions configured to anneal with adapter regions of amplicon target molecules and/or microbial community-associated fragments, sequencing adapter regions, and sequencing indexing regions,
wherein the normalized combined library is usable for simultaneous amplicon-associated sequencing and microbial community-associated sequencing, and
wherein generating the normalized mixture comprises adjusting a concentration ratio between the set of amplicon target molecules and the set of microbial community-associated fragments, based on a desired ratio of amplicon-associated sequencing reads and microbial community-associated sequencing reads.

2. The method of claim 1, further comprising performing an additional normalization process on the set of sequencing-ready molecules, wherein performing the additional normalization process comprises:
diluting magnetic beads to a desired ratio;
generating a set of bead-molecule compounds based on a binding process with the magnetic beads and the set of sequencing-ready molecules;
attaching the set of bead-molecule compounds to an interface plate of a magnetic field system; and
separating the set of bead-molecule compounds from the interface plate.

3. The method of claim 1, further comprising, prior to generating the set of amplicon target molecules, performing at least one of:
transforming microbial RNA from the sample into cDNA for facilitating preparation of a normalized microbial RNA library for sequencing; and
transforming cDNA into double stranded cDNA for facilitating preparation of a normalized library for sequencing.

4. The method of Claim 1, wherein the set of amplicon-generation primers includes first primers corresponding to a first primer type configuration including 5'-EXTERNAL DEFINED SEQUENCE (XXXXXXX) -EXTERNAL ADAPTER- SPACER-TARGET SEQUENCE UPST-3', and second primers corresponding to a second primer type configuration including 5'-EXTERNAL DEFINED SEQUENCE (YYYYYYY)-EXTERNAL ADAPTER-SPACER-TARGET SEQUENCE DWNST-3'.

5. The method of Claim 1, wherein the set of ligation adapter molecules comprises first ligation adapter molecules corresponding to a first ligation adapter molecule type of a configuration including 3' EXTERNAL DEFINED SEQUENCE (XXXXXXX)-ADAPTER A-BRIDGE-PHOSPHATE 5'; and second ligation adapter molecules corresponding to a second ligation adapter molecule type of a configuration including 5' EXTERNAL DEFINED SEQUENCE (YYYYYYY)- ADAPTER_B-BRIDGE- ADENINE 3'.

6. The method of Claim 1, wherein the set of normalization-based primers include a first type of primer pair including 5'-EXTERNAL ADAPTER-ADAPTER A1-3' and 5'-EXTERNAL ADAPTER-ADAPTER A2-3' and/or a second type of primer pair including 5'-EXTERNAL ADAPTER-ADAPTER M1-3' and 5'-EXTERNAL ADAPTER-ADAPTER M2-3' in which ADAPTER-A1 and ADAPTER-A2 regions enable amplification of the amplicon target molecules and ADAPTER-M1 and ADAPTER-M2 enable amplification of the microbial community-associated fragments.

7. The method of Claim 1, wherein the amplicon-generation primers or the ligation adapter molecules include one or more unique molecular identifier (UMI) regions in which each UMI region comprises a set of random "N" bases, wherein each random "N" base is selected from any one of an "A" base, a "G" base, a "T" base, and a "C" base, and has a sequence length in between 2 to 20 bases.

## Patentansprüche

1. Verfahren zur Herstellung einer normierten kombinierten Bibliothek, wobei das Verfahren umfasst:
Erzeugen einer Menge von Amplikon-Zielmolekülen auf der Basis eines ersten Polymerase-Kettenreaktions(PCR)verfahrens mit Zielmolekülen von wenigstens einer Probe, die Mikroorganismen aus der Mikrobengemeinschaft umfasst, wobei die Zielmoleküle der Menge an Targets entsprechen, die mit einem oder mehreren Bereichen von Amplikonerzeugungsprimern assoziiert sind, wobei die Menge von Amplikon-Zielmolekülen externe definierte Sequenzbereiche und Adapterbereiche umfasst;
Erzeugen einer Menge von mit der Mikrobengemeinschaft assoziierten Fragmenten auf der Basis der Verarbeitung von Gesamtnucleinsäuren aus der wenigstens einen Probe, wobei die Menge von mit der Mikrobengemeinschaft assoziierten Fragmenten wenigstens eine von einer Menge von mikrobiellen DNA-Fragmenten und einer Menge von mikrobiellen RNA-Fragmenten umfasst, und Erzeugen einer Menge von ligierten, mit der Mikrobengemeinschaft assoziierten Fragmenten auf der Basis eines Ligationsvorgangs mit der Menge der mit der Mikrobengemeinschaft assoziierten Fragmente und einer Menge von Ligationsadaptermolekülen, wobei die Menge von Ligationsadaptermolekülen externe definierte Sequenzbereiche und Adapterbereiche, die mit der Sequenzierung assoziiert sind, umfasst und wobei die Menge von ligierten, mit der Mikrobengemeinschaft assoziierten Fragmenten externe definierte Sequenzbereiche und Adapterbereiche umfasst;
Erzeugen eines normierten Gemischs auf der Basis eines zweiten PCR-Verfahrens mit der Menge der Amplikon-Zielmoleküle, der Menge der mit der Mikrobengemeinschaft assoziierten Fragmente und einer Menge von Primern auf Normierungsbasis, wobei die Menge der Primer auf Normierungsbasis eine Menge von Amplikon-assoziierten Primern, die mit Amplikon-Zielmolekülen assoziiert sind, und eine Menge von mit der Mikrobengemeinschaft assoziierten Primern, die mit mit der Mikrobengemeinschaft assoziierten Fragmenten assoziiert sind, umfasst und wobei die Menge der Primer auf Normierungsbasis externe definierte Sequenzbereiche zum Hybridisieren mit der Menge der Amplikon-Zielmoleküle und der Menge der mit der Mikrobengemeinschaft assoziierten Fragmente umfasst, wobei eine definierte Anzahl von Molekülen von Primern auf Normierungsbasis hinzugefügt wird, um zu gewährleisten, dass alle hinzugefügten Primer auf Normierungsbasis vollständig verbraucht sind; und
Erzeugen der normierten kombinierten Bibliothek, die eine Menge von sequenzierungsbereiten Molekülen auf der Basis eines dritten PCR-Verfahrens mit dem normierten Gemisch und eine Menge von sequenzierungsbasierten Primern, die mit der Sequenzierung assoziierte Adapterbereiche umfassen, umfasst, wobei die Menge der sequenzierungsbereiten Moleküle mit der Menge von Targets und den mit der Mikrobengemeinschaft assoziierten Fragmenten assoziiert ist, wobei die Menge der sequenzierungsbasierten Primer Adapterbereiche, die so konfiguriert sind, dass sie mit Adapterbereichen von Amplikon-Zielmolekülen und/oder mit der Mikrobengemeinschaft assoziierten Fragmenten hybridisieren können, Sequenzierungsadapterbereiche und Sequenzierungsindexbereiche umfasst,
wobei die normierte kombinierte Bibliothek für gleichzeitige amplikonassoziierte Sequenzierung und mit der Mikrobengemeinschaft assoziierte Sequenzierung geeignet ist und
wobei das Erzeugen des normierten Gemischs das Einstellen eines Konzentrationsverhältnisses zwischen der Menge der Amplikon-Zielmoleküle und der Menge der mit der Mikrobengemeinschaft assoziierten Fragmente auf der Basis eines gewünschten Verhältnisses von Ablesungen der amplikonassoziierten Sequenzierung und Ablesungen der mit der Mikrobengemeinschaft assoziierten Sequenzierung umfasst.

2. Verfahren gemäß Anspruch 1, weiterhin umfassend das Durchführen eines zusätzlichen Normierungsverfahrens mit der Menge der sequenzierungsbereiten Moleküle, wobei das Durchführen des zusätzlichen Normierungsverfahrens Folgendes umfasst:
das Verdünnen von Magnetkügelchen auf ein gewünschtes Verhältnis;
das Erzeugen einer Menge von Kügelchen-Molekül-Konjugaten auf der Basis eines Bindungsvorgangs mit den Magnetkügelchen und der Menge der sequenzierungsbereiten Moleküle;
Binden der Menge von Kügelchen-Molekül-Konjugaten an eine Schnittstellenplatte eines Magnetfeldsystems; und
Abtrennen der Menge von Kügelchen-Molekül-Konjugaten von der Schnittstellenplatte.

3. Verfahren gemäß Anspruch 1, weiterhin umfassend das Durchführen wenigstens eines der folgenden Schritte vor dem Erzeugen der Menge von Amplikon-Zielmolekülen:
Transformieren mikrobieller RNA aus der Probe zu cDNA zur Erleichterung der Herstellung einer normierten mikrobiellen RNA-Bibliothek zum Sequenzieren; und
Transformieren von cDNA zu doppelsträngiger cDNA zur Erleichterung der Herstellung einer normierten Bibliothek zum Sequenzieren.

4. Verfahren gemäß Anspruch 1, wobei die Menge der Amplikonerzeugungsprimer erste Primer, die einer ersten Primertypkonfiguration entsprechen, welche 5'-EXTERNE DEFINIERTE SEQUENZ (XXXXXXX)-EXTERNER ADAPTER-SPACER-ZIELSEQUENZ-UPST-3' umfasst, und zweite Primer, die einer zweiten Primertypkonfiguration entsprechen, welche 5'-EXTERNE DEFINIERTE SEQUENZ (YYYYYYY)-EXTERNER ADAPTER-SPACER-ZIELSEQUENZ-DWNST-3' umfasst, umfasst.

5. Verfahren gemäß Anspruch 1, wobei die Menge der Ligationsadaptermoleküle erste Ligationsadaptermoleküle, die einem ersten Ligationsadaptermolekültyp einer Konfiguration entsprechen, welche 3'-EXTERNE DEFINIERTE SEQUENZ (XXXXXXX)-ADAPTER_A-BRÜCKE-PHOSPHAT-5' umfasst, und zweite Ligationsadaptermoleküle, die einem zweiten Ligationsadaptermolekültyp einer Konfiguration entsprechen, welche 5'-EXTERNE DEFINIERTE SEQUENZ (YYYYYYY)-ADAPTER_B-BRÜCKE-ADENIN-3' umfasst, umfasst.

6. Verfahren gemäß Anspruch 1, wobei die Menge der normierungsbasierten Primer einen ersten Typ von Primerpaar, das 5'-EXTERNER ADAPTER-ADAPTER A1-3' und 5'-EXTERNER ADAPTER-ADAPTER A2-3' umfasst, und/oder einen zweiten Typ von Primerpaar, das 5'-EXTERNER ADAPTER-ADAPTER M1-3' und 5'-EXTERNER ADAPTER-ADAPTER M2-3' umfasst, umfasst, wobei die Bereiche ADAPTER-A1 und ADAPTER-A2 die Amplifikation der Amplikon-Zielmoleküle ermöglichen und ADAPTER-M1 und ADAPTER-M2 die Amplifikation der mit der Mikrobengemeinschaft assoziierten Fragmente ermöglichen.

7. Verfahren gemäß Anspruch 1, wobei die Amplikonerzeugungsprimer oder die Ligationsadaptermoleküle einen oder mehrere UMI-Bereiche (UMI: eindeutige molekulare Identifikatoren) umfassen, wobei jeder UMI-Bereich eine Menge von zufälligen "N"-Basen umfasst, wobei jede zufällige "N"-Base beliebig aus einer "A"-Base, einer "G"-Base, einer "T"-Base und einer "C"-Base ausgewählt ist und eine Sequenzlänge von 2 bis 20 Basen aufweist.

## Revendications

1. Procédé de préparation d'une banque combinée normalisée, le procédé comprenant :
la génération d'un ensemble de molécules cibles d'amplicon d'après un premier processus d'amplification en chaîne par polymérase (PCR) avec des molécules cibles d'au moins un échantillon comportant des micro-organismes de la communauté microbienne, dans lequel les molécules cibles correspondent à l'ensemble de cibles associées à une ou plusieurs régions d'amorces de génération d'amplicon, dans lequel l'ensemble de molécules cibles d'amplicon comprend des régions de séquence définies externes et des régions d'adaptateur ;
la génération d'un ensemble de fragments associés à la communauté microbienne d'après le traitement d'acides nucléiques totaux à partir de l'au moins un échantillon, dans lequel l'ensemble de fragments associés à la communauté microbienne comprend au moins l'un d'un ensemble de fragments d'ADN microbien et d'un ensemble de fragments d'ARN microbien, et la génération d'un ensemble de fragments ligaturés associés à la communauté microbienne d'après un processus de ligature avec l'ensemble de fragments associés à la communauté microbienne et un ensemble de molécules d'adaptateur de ligature, dans lequel l'ensemble de molécules d'adaptateur de ligature comprend des régions de séquence définies externes et des régions d'adaptateur associées au séquençage, et dans lequel l'ensemble de fragments ligaturés associés à la communauté microbienne comprend des régions de séquence définies externes et des régions d'adaptateur ;
la génération d'un mélange normalisé d'après un deuxième processus PCR avec l'ensemble de molécules cibles d'amplicon, l'ensemble de fragments associés à la communauté microbienne et un ensemble d'amorces basées sur la normalisation, dans lequel l'ensemble d'amorces basées sur la normalisation comporte un ensemble d'amorces associées à un amplicon associées à des molécules cibles d'amplicon et un ensemble d'amorces associées à la communauté microbienne associées à des fragments associés à la communauté microbienne, et dans lequel l'ensemble d'amorces basées sur la normalisation comprend des régions de séquence définies externes pour un annelage de l'ensemble de molécules cibles d'amplicon et l'ensemble de fragments associés à la communauté microbienne, dans lequel un nombre défini de molécules d'amorces basées sur la normalisation est ajouté pour garantir que toutes les amorces basées sur la normalisation ajoutées sont totalement utilisées ; et
la génération de la banque combinée normalisée comprenant un ensemble de molécules prêtes pour le séquençage d'après un troisième processus PCR avec le mélange normalisé et un ensemble d'amorces basées sur le séquençage comprenant des régions d'adaptateur associées au séquençage, dans lequel l'ensemble de molécules prêtes pour le séquençage est associé à l'ensemble de cibles et aux fragments associés à la communauté microbienne, dans lequel l'ensemble d'amorces basées sur le séquençage comprend des régions d'adaptateur configurées pour un annelage avec des régions d'adaptateur de molécules cibles d'amplicon et/ou de fragments associés à la communauté microbienne, de régions d'adaptateur de séquençage, et de régions d'indexation de séquençage,
dans lequel la banque combinée normalisée peut être utilisée pour un séquençage associé à un amplicon et un séquençage associé à la communauté microbienne simultanés, et
dans lequel la génération du mélange normalisé comprend l'ajustement d'un rapport de concentration entre l'ensemble de molécules cibles d'amplicon et l'ensemble de fragments associés à la communauté microbienne, d'après un rapport souhaité entre des lectures de séquençage associées à un amplicon et des lectures de séquençage associées à la communauté microbienne.

2. Procédé selon la revendication 1, comprenant en outre la réalisation d'un processus de normalisation supplémentaire sur l'ensemble de molécules prêtes pour le séquençage, dans lequel la réalisation du processus de normalisation supplémentaire comprend :
la dilution de billes magnétiques à un rapport souhaité ;
la génération d'un ensemble de composés billes-molécules d'après un processus de liaison avec les billes magnétiques et l'ensemble de molécules prêtes pour le séquençage ;
la fixation de l'ensemble de composés billes-molécules à une plaque d'interface d'un système de champ magnétique ; et
la séparation de l'ensemble de composés billes-molécules d'avec la plaque d'interface.

3. Procédé selon la revendication 1, comprenant en outre, avant la génération de l'ensemble de molécules cibles d'amplicon, la réalisation d'au moins l'une parmi :
la transformation d'ARN microbien de l'échantillon en ADNc pour faciliter la préparation d'une banque normalisée d'ARN microbien pour le séquençage ; et
la transformation d'ADNc en ADNc double brin pour faciliter la préparation d'une banque normalisée pour le séquençage.

4. Procédé selon la revendication 1, dans lequel l'ensemble d'amorces de génération d'amplicon comporte des premières amorces correspondant à une première configuration de type amorce comportant S'-SEQUENCE DEFINIE EXTERNE (XXXXXXX)-ADAPTATEUR EXTERNE-ESPACEUR-SEQUENCE CIBLE AMONT-3', et des secondes amorces correspondant à une seconde configuration de type amorce comportant 5'-SEQUENCE DEFINIE EXTERNE (YYYYYYY)-ADAPTATEUR EXTERNE-ESPACEUR-SEQUENCE CIBLE AVAL-3'.

5. Procédé selon la revendication 1, dans lequel l'ensemble de molécules d'adaptateur de ligature comprend des premières molécules d'adaptateur de ligature correspondant à un premier type de molécule d'adaptateur de ligature d'une configuration comportant 3'-SEQUENCE DEFINIE EXTERNE (XXXXXXX)-ADAPTATEUR A-PONT-PHOSPHATE 5' ; et des secondes molécules d'adaptateur de ligature correspondant à un second type de molécule d'adaptateur de ligature d'une configuration comportant 5' SEQUENCE DEFINIE EXTERNE (YYYYYYY)- ADAPTATEUR_B-PONT-ADENINE 3'.

6. Procédé selon la revendication 1, dans lequel l'ensemble d'amorces basées sur la normalisation comporte un premier type de paire d'amorces comportant 5'-ADAPTATEUR EXTERNE-ADAPTATEUR A1-3' et 5'-ADAPTATEUR EXTERNE-ADAPTATEUR A2-3' et/ou un second type de paire d'amorces comportant 5'-ADAPTATEUR EXTERNE-ADAPTATEUR M1-3' et 5'-ADAPTATEUR EXTERNE-ADAPTATEUR M2-3' dans lequel des régions ADAPTATEUR-A1 et ADAPTATEUR-A2 permettent une amplification des molécules cibles d'amplicon et ADAPTATEUR-M1 et ADAPTATEUR-M2 permettent une amplification des fragments associés à la communauté microbienne.

7. Procédé selon la revendication 1, dans lequel les amorces de génération d'amplicon ou les molécules d'adaptateur de ligature comportent une ou plusieurs régions d'identifiant moléculaire unique (UMI) dans lesquelles chaque région UMI comprend un ensemble de bases « N » aléatoires, dans lequel chaque base « N » aléatoire est choisie parmi une quelconque d'une base « A », d'une base « G », d'une base « T » et d'une base « C », et a une longueur de séquence entre 2 et 20 bases.
